(19) Europäisches Patentamt / European Patent Office / Office européen des brevets

(11) **EP 4 190 354 A1**

(12) **EUROPEAN PATENT APPLICATION**
published in accordance with Art. 153(4) EPC

(43) Date of publication:
07.06.2023 Bulletin 2023/23

(21) Application number: 21850476.9

(22) Date of filing: 27.07.2021

(51) International Patent Classification (IPC):
*A61K 39/395* (2006.01)  *C07K 16/00* (2006.01)
*A61K 9/08* (2006.01)  *A61M 5/32* (2006.01)
*C12N 15/13* (2006.01)

(52) Cooperative Patent Classification (CPC):
A61K 9/08; A61K 39/395; A61M 5/32; C07K 16/00

(86) International application number:
PCT/JP2021/027663

(87) International publication number:
WO 2022/025030 (03.02.2022 Gazette 2022/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.07.2020 JP 2020127605

(71) Applicants:
• CHUGAI SEIYAKU KABUSHIKI KAISHA
Tokyo 115-8543 (JP)
• F. Hoffmann-La Roche AG
4070 Basel (CH)

(72) Inventors:
• FUKUDA, Masakazu
Tokyo 115-8543 (JP)
• YAMASHITA, Shogo
Tokyo 115-8543 (JP)
• YAMANAKA, Yuji
Tokyo 115-8543 (JP)
• MUELLER, Robert
Basel CH4070 (CH)
• YANG, Kewei
Basel CH4070 (CH)

(74) Representative: **Vossius & Partner
Patentanwälte Rechtsanwälte mbB
Siebertstraße 3
81675 München (DE)**

Remarks:
The complete document including Reference Table(s) and the Sequence Listing(s) can be downloaded from the EPO website

(54) **PREFILLED SYRINGE PREPARATION WITH NEEDLE, PROVIDED WITH NEEDLE SHIELD AND INCLUDING NOVEL MODIFIED ANTIBODY**

(57) An object of the present disclosure is to provide a staked-in needle prefilled syringe formulation containing an antibody engineered by a mutation to histidine, which reduces the difficulty in pushing out a drug and the risk of causing clogging in a needle. The staked-in needle prefilled syringe formulation can be produced by a simple method on an industrial scale.

The present prefilled syringe formulation is characterized by having an elastomeric needle shield having a low zinc leaching capacity.

Fig. 2

EP 4 190 354 A1

**Description**

TECHNICAL FIELD

The present disclosure relates to a staked-in needle prefilled syringe formulation with a needle cap for protecting the needle. The present disclosure particularly relates to reducing undesirable interactions between a needle shield and a drug solution containing an engineered antibody that is susceptible to zinc leaching.

BACKGROUND ART

In recent years, various antibody formulations have been developed and put into practical use. Many antibody formulations have been used as intravenous injectable formulations. However, with the needs of the medical field, there is an increasing demand for development of antibody-containing formulations as self-injectable subcutaneous injection formulations. In particular, there is a high demand for development of solution formulations encased in prefilled syringes due to their convenience.

In designing an antibody-containing formulation for subcutaneous injection, a high concentration antibody is needed in the dosing solution since a large amount of antibody is injected per dose while the injectable amount in a subcutaneous injection is generally limited.

In recent years as a prefilled syringe formulation for self-injection, a staked-in needle prefilled syringe in which a needle is attached to the tip of the syringe body has become used in the medical field. In such a prefilled syringe formulation, a pharmaceutical substance is provided inside the syringe in a dissolved form so that it can be administered immediately, and the syringe is produced and shipped with a needle attached to the body. This type of prefilled syringe is typically transported and stored with the injection needle being protected by a needle cap. The needle cap is removed when the formulation is used. The needle cap generally has a needle shield portion that is an inner elastic portion adjoining and surrounding the needle and a rigid needle shield portion that is an outer rigid portion made of thermosetting plastic material or the like.

The needle cap prevents drug leakage and contamination of foreign substances by sealing the needle tip. However, clogging may occur in or near the needle, depending on the drug. As a result, the drug may not be properly pushed out of the syringe. Such cases have a risk that an appropriate dosage cannot be administered, thus clogging of the needle is undesirable. Particularly, when the prefilled syringe formulation is used in home care, such a state of the formulation may cause delayed treatment because the formulation is unusable. Therefore, in the development of a staked-in needle prefilled syringe formulation, needle clogging has been an important technical issue.

The main cause of needle clogging is believed to be the drying of the drug solution in the needle during long-term storage or after removing the needle cap in use. In particular, high-concentrated protein formulations have a high viscosity of the drug solution, which is likely to cause needle clogging due to drying. Further, protein aggregation or gelation makes it difficult or impossible to push out the drug solution, and when drying of the drug solution is added, needle clogging becomes more likely to occur.

When a staked-in needle prefilled syringe solution formulation containing a high concentration antibody formulation is left in a dry state for a long period without any enclosure such as a film, or after being removed from the enclosure, the solution formulation in the needle is dried and the needle clogging occurs with use of a needle cap made of breathable material. Patent Literature 1 discloses a prefilled syringe formulation characterized by having a needle cap made of a low water vapor permeability-material to solve such clogging due to drying.

Patent Literature 2 describes improving the needle shield to prevent clogging of the needle in a staked-in needle prefilled syringe or the like. This literature describes making the needle shield material be low zinc (Zn) leaching and using a needle shield material of water vapor tight to reduce the water vapor permeability of the needle shield and prevent an increase of viscosity of the pharmaceutical substance solution in the needle by zinc ions that can leach from the material (elastomer) of the needle shield. Specifically, it has been confirmed in Examples that the clogging rate has been improved by using a material of the needle shield having a low water vapor permeability.

In this way, the problem to be solved in the prior art was to prevent clogging of the needle mainly due to drying.

CITATION LIST

PATENT LITERATURE

[Patent Literature 1] Domestic Re-publication of PCT International Publication for Patent Application No. 2016-068333

[Patent Literature 2] National Publication of International Patent Application No. 2017-538463 (WO 2016/066821)

SUMMARY OF INVENTION

TECHNICAL PROBLEM

The present inventors have found that pushing out the drug solution from the needle in the staked-in needle prefilled syringe containing the high-concentration antibody formulation may become difficult during long-term storage, even if the formulation is not in a dry state and the needle clogging can occur. The present inventors have further considered that the problem of difficulty in pushing out the drug solution is caused by the leaching of zinc ions into the drug solution from the needle shield protecting the needle tip of the syringe which leads to gelation of the drug solution in the needle tip to increase the viscosity.

The present inventors have found that the degree of viscosity increase in the presence of zinc ions varies depending on the antibody that is an active ingredient. They have also confirmed that the leaching amount of zinc ions into the drug solution differs depending on the material of the needle shield. Furthermore, they have confirmed that the leaching amount of zinc ions varies depending on the buffer composition of the drug solution.

The present inventors have identified characteristics of antibodies that are particularly susceptible to zinc ions. For example, antibodies containing histidine residues due to certain characteristics, such as recycling antibodies or low pI antibodies, have been found to have enhanced interaction with zinc ions, which may cause gelation by zinc ions.

The "recycling antibody" is an antibody that is incorporated into a cell in a state bound to an antigen and released from the cell in a state not bound to the antigen. The recycling antibody is characterized by having a substitution of at least one amino acid of the antibody with histidine or an insertion of at least one histidine. (WO 2009125825, US 20110111406).

The "low pI antibody" is an antibody whose half-life in plasma is suppressed by engineering of an amino acid residue that can be exposed to the surface of a complementarity determining region (CDR) of the antibody (WO 2009041643, US 20100239577).

In general, the quality of a material of the container of the pharmaceutical product must meet a predetermined standard. Furthermore, it is important to verify the compatibility of the container material with individual pharmaceutical products to ensure the quality of the pharmaceutical product. Thus, it is important that the most suitable material for the needle shield of the staked-in needle prefilled syringe formulation is selected from materials that meet a predetermined standard, with consideration for the compatibility with the drug solution to fill the syringe.

Focusing on the interaction between the needle shield and the antibody solution in the staked-in needle prefilled syringe antibody formulations, an object of the present disclosure is to provide a means of preventing viscosity increase and/or clogging in or near the needle so as not to cause problems in pushing out of the drug solution after storage. Another object of the present disclosure is to provide a staked-in needle prefilled syringe antibody formulation for an antibody solution that is susceptible to the leaching of zinc.

SOLUTION TO PROBLEM

The present inventors have newly found a group of antibodies suitable for use with a needle shield of low zinc leaching capacity. Furthermore, the present inventors have identified a needle shield having a low zinc leaching capacity by evaluating the zinc leaching amount of various needle shield materials. Selecting the needle shield material having a low zinc leaching capacity to the antibody-containing solution can suppress increasing in viscosity of a drug solution and prevent clogging of the needle, even when using an antibody solution susceptible to zinc.

More specifically, the present disclosure provides the following.

[1] A prefilled syringe formulation including: a staked-in needle syringe filled with a drug solution; and an elastomeric needle shield, wherein

the drug solution contains one or more antibodies,
the one or more antibodies have a total number of histidine, aspartic acid, and glutamic acid present in a CDR region of 6 or more, and
the elastomeric needle shield has a low zinc leaching capacity to an antibody-containing solution.

In the present disclosure, the one or more antibodies, for example, in the prefilled syringe formulation according to [1] above, or in other aspects of the present disclosure, can optionally have one or more following properties respectively or in combination.

[1-1] The antibody has one or more histidine residue pairs that are adjacent to each other within three residues in a CDR region.

[1-2] The antibody has one histidine residue pair described above.

[1-3] The antibody binds to an antigen with higher binding activity at a neutral pH than at an acidic pH.

[1-4] The antibody according to [1-3] above has a value of KD (pH 5.8)/(pH 7.4), a ratio of KD at pH 5.8 to KD at pH 7.4 for an antigen, of 2 or more; or

[1-5] the antibody has a value of KD (pH 5.8)/(pH 7.4) of 10 or more; or [1-6] the antibody has a value of KD (pH 5.8)/(pH 7.4) of 40 or more.

[1-7] The antibody has a substitution of at least one amino acid with histidine or an insertion of at least one histidine.

[1-8] The antibody has an antagonist activity.

[1-9] The antibody binds to a membrane antigen or a soluble antigen.

[1-10] The antibody is an antibody in which a charge of at least one amino acid residue that can be exposed to a surface of a CDR region is engineered.

[1-11] The antibody is selected from the group consisting of an anti-IL-6 receptor antibody, an anti-IL-8 antibody, and an anti-myostatin antibody.

[1-12] The antibody is an anti-IL-6 receptor antibody.

[1-13] The antibody is an anti-IL-8 antibody.

[1-14] The antibody is an anti-myostatin antibody.

[1-15] The antibody is selected from the group consisting of satralizumab (RG6168), mAb2, and RG6237.

[1-16] The antibody is satralizumab (RG6168).

[1-17] The antibody is mAb2 described herein.

[1-18] The antibody is RG6237.

[2] The prefilled syringe formulation according to any one of [1] and [1-1] to [1-18], wherein the low zinc leaching capacity is evaluated by a zinc leaching rate at 5°C per surface area of the needle shield in a buffer at pH 6.0, containing 20 mM histidine, 150 mM arginine, aspartic acid (q.s.), and 0.5 mg/mL poloxamer 188.

In the present disclosure, the low zinc leaching capacity, for example, in the prefilled syringe formulation according to [2] above, or in other aspects of the present disclosure, can optionally have one or more following properties respectively or in combination.

[2-1] The zinc leaching rate is 7 ng/mm$^2$/day or less.

[2-2] The zinc leaching rate is 0.88 ng/mm$^2$/day or less.

[2-3] The low zinc leaching capacity is evaluated by an expected zinc concentration after 2 years at 5°C in a needle bevel, obtained from a zinc leaching rate at 5°C per surface area of the needle shield.

[2-4] The expected zinc concentration is 5 mg/mL or less.

[2-5] The expected zinc concentration is 0.63 mg/mL or less.

[3] The prefilled syringe formulation according to any one of [1], [1-1] to [1-18], [2], and [2-1] to [2-5], wherein a material of the needle shield is selected from the group consisting of a thermoplastic elastomer and a thermosetting elastomer.

In the present disclosure, the material of the elastomeric needle shield, for example, in the prefilled syringe formulation according to [3] above, or in other aspects of the present disclosure, can optionally have one or more following properties respectively or in combination.

[3-1] The material of the needle shield is selected from the group consisting of an ethylene propylene diene type thermoplastic elastomer and a chlorobutyl type thermosetting elastomer.

[3-2] The material of the needle shield is selected from the group consisting of BD260, Stelmi 8550, Sumitomo P-101A.

[4] The prefilled syringe formulation according to any one of [1], [1-1] to [1-18], [2], [2-1] to [2-5], [3] and [3-1] to [3-2], wherein the solution of the antibody has a viscosity of 2 to 100 mPa·s (pascal seconds) at 25°C.

In the present disclosure, the concentration and/or viscosity of the solution of the antibody, for example, in the prefilled syringe formulation according to [4] above, or in other aspects of the present disclosure, can optionally have one or more following properties respectively or in combination.

[4-1] The solution of the antibody has a viscosity of 2 to 30 mPa·s (pascal seconds) at 25°C.

[4-2] The concentration of the antibody in the solution is 50 to 300 mg/mL, and the solution of the antibody has a viscosity of 2 to 30 mPa·s (pascal seconds) at 25°C.

[5] A method of suppressing a viscosity increase and/or a needle clogging of a prefilled syringe, including: a staked-in needle syringe filled with a drug solution; and an elastomeric needle shield, the method characterized in that

the drug solution contains one or more antibodies having a total number of histidine, aspartic acid, and glutamic acid present in a CDR region of 6 or more,
the method includes selecting a material of the needle shield so that the needle shield has a low zinc leaching capacity to the drug solution, and
the material of the needle shield is selected from the group consisting of a thermoplastic elastomer and a thermosetting elastomer.

In the present disclosure, the one or more antibodies, for example, in the method according to [5] above, or in other aspects of the present disclosure, can optionally have one or more properties as described in [1-1] to [1-18] above, respectively or in combination.

[6] The method according to [5], wherein the low zinc leaching capacity is evaluated by a zinc leaching rate at 5°C per surface area of the needle shield in a buffer at pH 6.0, containing 20 mM histidine, 150 mM arginine, aspartic acid (q.s.), and 0.5 mg/mL poloxamer 188.

In the present disclosure, the low zinc leaching capacity, for example, in the method according to [6] above, or in other aspects of the present disclosure, can optionally have one or more properties as described in [2-1] to [2-5] above, respectively or in combination.

[7] The method according to [5] or [6], wherein the solution of the antibody has a viscosity of 2 to 100 mPa·s (pascal seconds) at 25°C.

In the present disclosure, the concentration and/or viscosity of the solution of the antibody, for example, in the method according to [7] above, or in other aspects of the present disclosure, can optionally have one or more properties as described in [4-1] to [4-2] above, respectively or in combination.

[8] A syringe including: a longitudinal body having an interior where an antibody-containing solution is placed; a needle connected to one end in a longitudinal direction of the longitudinal body; and a needle cap encasing the needle, wherein

the needle cap has an elastomeric needle shield, and
the needle shield has a low zinc leaching capacity to a solution containing one or more antibodies having a total number of histidine, aspartic acid, and glutamic acid present in a CDR region of 6 or more.

In the present disclosure, the one or more antibodies, for example, in the syringe according to [8] above, or in other aspects of the present disclosure, can optionally have one or more properties as described in [1-1] to [1-18] above, respectively or in combination.

[9] The syringe according to [8], wherein the low zinc leaching capacity is evaluated by an expected zinc concentration after 2 years at 5°C in a needle bevel, obtained from a zinc leaching rate at 5°C per surface area of the needle shield.

In the present disclosure, the low zinc leaching capacity, for example, in the syringe according to [9] above, or in other aspects of the present disclosure, can optionally have one or more properties as described in [2-1] to [2-5] above, respectively or in combination.

[10] The syringe according to [8] or [9], wherein a material of the needle shield is selected from the group consisting of a thermoplastic elastomer and a thermosetting elastomer.

In the present disclosure, the material of the elastomeric needle shield, for example, in the syringe according to [10] above, or in other aspects of the present disclosure, can optionally have one or more properties as described in [3-1] or [3-2] above, respectively or in combination.

[11] A method for producing a staked-in needle syringe formulation, including:

providing a drug solution containing one or more antibodies having a total number of histidine, aspartic acid, and glutamic acid present in a CDR region of 6 or more;
providing a needle shield having a low zinc leaching capacity to the drug solution; and
filling a staked-in needle syringe having the needle shield with the drug solution, wherein
the needle shield has a zinc leaching rate at 5°C per surface area of 0.88 ng/mm$^2$/day or less to the drug solution.

In the present disclosure, the one or more antibodies, for example, in the method according to [11] above, or in other aspects of the present disclosure, can optionally have one or more properties as described in [1-1] to [1-18] above, respectively or in combination.

Also in the present disclosure, the low zinc leaching capacity, for example, in the method according to [11] above, or in other aspects of the present disclosure, can optionally have one or more properties as described in [2] and [2-1] to [2-5] above, respectively or in combination.

[12] The method according to [11], wherein the drug solution containing the antibody has a viscosity of 2 to 100 mPa·s (pascal seconds) at 25°C.

In the present disclosure, the concentration and/or viscosity of the drug solution containing the antibody, for example, in the method according to [12] above, or in other aspects of the present disclosure, can optionally have one or more properties as described in [4-1] to [4-2] above, respectively or in combination.

[13] A method of selecting a needle shield, the method including: providing two or more elastomeric needle shields; evaluating the needle shield for zinc leaching capacity in a predetermined buffer for an antibody solution formulation; and selecting a needle shield having a lower zinc leaching capacity, wherein the evaluation of zinc leaching capacity is performed by calculating the leaching rate of zinc ions per unit surface area of the needle shield to the predetermined buffer and/or determining an expected zinc concentration in the needle.

[14] The method according to [13], wherein the zinc leaching capacity is evaluated by a zinc leaching rate at 5°C per surface area of the needle shield in a buffer containing about 20 mM histidine, pH 6.0, prepared using aspartic acid as a pH adjuster.

[15] The method according to [13], wherein the low zinc leaching capacity is evaluated by zinc leaching rate at 5°C per surface area of the needle shield in a buffer at pH 6.0, containing 20 mM histidine, 150 mM arginine, aspartic acid (q.s.), and 0.5 mg/mL poloxamer 188.

In the present disclosure, the low zinc leaching capacity, for example, in the method according to [13] to [15] above, or in other aspects of the present disclosure, can optionally have one or more properties as described in [2-1] to [2-5] above, respectively or in combination.

[16] A method for selecting a needle shield for a staked-in needle prefilled syringe formulation to be filled with a solution containing one or more antibodies having a total number of histidine, aspartic acid, and glutamic acid present in a CDR region of 6 or more, the method including:

> providing two or more elastomeric needle shields;
> evaluating the needle shield for zinc leaching capacity in a buffer at pH 6.0, containing 20 mM histidine, 150 mM arginine, aspartic acid (q.s.), and 0.5 mg/mL poloxamer 188; and selecting a needle shield having a lower zinc leaching capacity.

In the present disclosure, the one or more antibodies, for example, in the method according to [16] above, or in other aspects of the present disclosure, can optionally have one or more properties as described in [1-1] to [1-18] above, respectively or in combination.

[17] The method according to [16], wherein the low zinc leaching capacity is evaluated by a zinc leaching rate at 5°C per surface area of the needle shield.

> [17-1] The method according to [17], wherein a zinc leaching rate at 5°C per surface area of the needle shield is 7 ng/mm$^2$/day or less.
> [17-2] The method according to [17], wherein a zinc leaching rate at 5°C per surface area of the needle shield is 0.88 ng/mm$^2$/day or less.

[18] The method according to [16] or [17], wherein the low zinc leaching capacity is evaluated by an expected zinc concentration after 2 years at 5°C in a needle bevel, obtained from a zinc leaching rate at 5°C per surface area of the needle shield.

> [18-1] The method according to [18], wherein the expected zinc concentration is 5 mg/mL or less.
> [18-2] The method according to [18], wherein the expected zinc concentration is 0.63 mg/mL or less.

[19] The method according to any one of [16], [17], [17-1] to [17-2], [18], [18-1] to [18-2], wherein the solution of the antibody has a viscosity of 2 to 100 mPa·s (pascal seconds) at 25°C.

> [19-1] The method according to [18], wherein the solution of the antibody has a viscosity of 2 to 30 mPa·s (pascal seconds) at 25°C.

[19-2] The method according to [18], wherein a concentration of the antibody in the solution is 50 to 300 mg/mL, and the solution of the antibody has a viscosity of 2 to 30 mPa·s (pascal seconds) at 25°C.

[20] A staked-in needle prefilled syringe filled with an antibody solution, wherein the staked-in needle prefilled syringe has a needle cap including a needle shield, and

(a) the antibody is satralizumab "RG6168", and the needle shield is made of Elastomer D (Sumitomo P-101A); or
(b) the antibody is mAb2 described herein, and the needle shield is made of Elastomer D (Sumitomo P-101A); or
(c) the antibody is anti-myostatin antibody "RG6237" and the needle shield is made of Elastomer D (Sumitomo P-101A) or BD260.

[20-1] The staked-in needle prefilled syringe filled with an antibody solution according to [20], wherein the needle shield is in direct contact with the injection needle, and a material of the needle shield has a low zinc leaching capacity to the antibody solution.
[20-2] The staked-in needle prefilled syringe filled with an antibody solution according to [20] or [20-1], wherein the antibody solution has a viscosity of 2 to 30 mPa·s (pascal seconds) at 25°C.
[20-3] The staked-in needle prefilled syringe filled with an antibody solution according to [20], [20-1], or [20-2], wherein a concentration of the antibody in the antibody solution is 50 to 300 mg/mL and the antibody solution has a viscosity of 2 to 30 mPa·s (pascal seconds) at 25°C.

ADVANTAGEOUS EFFECT OF INVENTION

The formulation of the present disclosure reduces the interaction between antibodies and zinc ions leached from a needle shield to an antibody-drug solution contained in a needle, thereby reducing the viscosity increase of the drug solution in a needle bevel of a staked-in needle prefilled syringe formulation and clogging of the needle.

BRIEF DESCRIPTION OF DRAWINGS

Figure 1 shows the concentration of zinc ions extracted from a cut elastomer (10 g) into a 20 mL of extraction solution. Water (neutral), 10 mM phosphoric acid solution (acidic), or 10 mM sodium hydroxide solution (alkaline) was used as the extraction solution. The extraction conditions were an incubation at 90°C for 3 days. An extraction solution subjected to incubation without any elastomer was used as a control.
Figure 2 is a schematic diagram showing the staked-in needle prefilled syringe, and the preparation of a sample and a control for the measurement of inductively coupled plasma mass spectrometry.
Figure 3 shows the zinc leaching amount per surface area ($M_{zinc}$($\mu$g/mm$^2$)) of the elastomer (polyisoprene type elastomer, Elastomer C described in Table 1) when stored at 5°C for 3, 7, and 14 days in Formulation No. 9 (20 mM histidine, 150 mM arginine, aspartic acid (q.s.), 0.5 mg/mL poloxamer 188, pH 6.0). The zinc leaching rate at 5°C per surface area of the elastomer ($k_{ext}$($\mu$g/mm$^2$/day)) was calculated from the slope to yield 0.0139 ($\mu$g/mm$^2$/day).
Figure 4 shows the viscosities of mAb solutions (180 mg/ml mAb, 20 mM histidine, 150 mM arginine, aspartic acid (q.s.), 0.5 mg/ml poloxamer 188, pH 6.0) when zinc concentration is 0, 2.5, 5, or 10 mg/ml, at 25°C, respectively.
Figure 5 shows the viscosities of mAb2 solution (180 mg/ml mAb2, 20 mM histidine, 150 mM arginine, aspartic acid (q.s.), 0.5 mg/ml poloxamer 188, pH 6.0) when zinc concentration is 0, 0.63, or 1.25 mg/mL, at 25°C, respectively.
Figure 6 is a photograph of gelation of the mAb2 solution (180 mg/mL mAb2, 20 mM histidine, 150 mM arginine, aspartic acid (q.s.), 0.5 mg/mL poloxamer 188, pH 6.0) caused by the addition of 10 mg/mL zinc.

DESCRIPTION OF EMBODIMENTS

Needle cap and staked-in needle syringe

As used in the present disclosure, the "needle cap" is for protecting a syringe needle of a staked-in needle prefilled syringe formulation and preventing drug leakage and contamination of foreign matters. The needle cap is attached to the syringe body to be removable in use. Furthermore, the needle cap is fitted closely to the syringe body or a connector portion of the injection needle and the syringe body to seal the syringe needle.
The needle cap consists of a "needle shield" that is in direct contact with the injection needle and a "rigid needle shield" that surrounds the needle shield to prevent needle damage or the like or to protect a user.

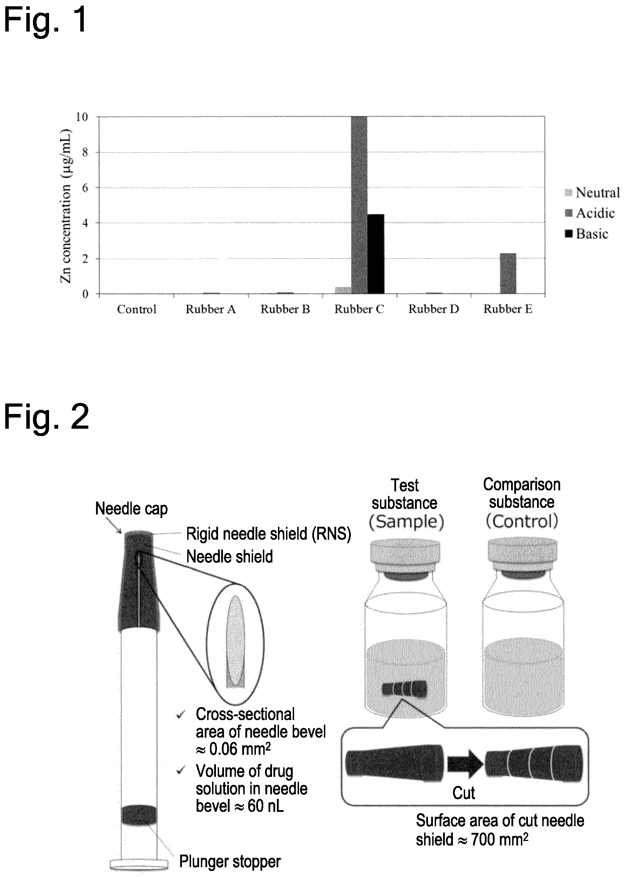

Figure 2 shows an example of a staked-in needle prefilled syringe having a needle cap.

Hereinafter, a staked-in needle prefilled syringe formulation having a needle cap including a needle shield according to an embodiment of the present disclosure is described.

A staked-in needle syringe according to the present disclosure has a syringe body having an interior which can be filled with a drug, an injection needle attached to a tip of the syringe body, and a needle cap removably attached to the syringe body. The needle cap is composed of a needle shield and a rigid needle shield, and the "needle shield" that is in direct contact with the injection needle is also in contact with the antibody solution in the injection needle. The present disclosure is characterized in that leaching of zinc ions from the needle shield into the antibody-containing solution in the needle is suppressed. Such configuration suppresses the increase of viscosity of the antibody solution in the needle after long-term storage and the difficulty in pushing out the drug solution. With antibody solutions having increased viscosity, if the needle cap is removed at the time of use and not put back immediately, the solution at the surface of the needle tip may be dried upon contact with the outside air, causing difficulty in pushing out the solution.

Zinc ions may be leached from the material composing the needle shield into the antibody solution. The leached zinc ions may interact with the antibody solution to increase the viscosity of the drug solution. Thus, in the present disclosure, it is preferred that the material of the needle shield is "an elastomer having a low zinc leaching capacity".

As used in the present disclosure, the "needle clogging" of a prefilled syringe is a phenomenon in which a drug solution dries and/or gels inside the needle, making it difficult to be pushed out when used. Thus, to prevent clogging of the needle during storage, the material of the needle cap is preferably a material having a low gas permeability, in particular, a low water vapor permeability.

The needle shield material of the present disclosure should have an elasticity that is removable from the syringe body and adherable to the syringe body. Furthermore, it is preferred that the material of the rigid needle shield and/or the needle shield composing the needle cap has a low water vapor permeability.

The presence or absence and/or the extent of zinc leaching from the needle shield can be determined by measuring the amount of zinc ions leached from the needle shield into an aqueous solution using, as appropriate, a known microanalysis method. For example, a test piece of a material of the needle shield is incubated in a predetermined buffer that can be used for an antibody formulation at a predetermined temperature for a predetermined time. Then, the leaching rate of zinc ions per unit surface area of the needle shield is calculated from the amount of zinc ions measured using inductively coupled plasma mass spectrometry (ICP-MS). The leaching rate of zinc ions can be used as an indicator. Detailed conditions for the analysis can be seen in the Examples section herein. The zinc leaching capacity described in the present disclosure is based on, unless otherwise specified, the method described in Example 2 herein.

For example, in the present disclosure, a low zinc leaching capacity refers to a zinc leaching capacity having a zinc leaching rate at 5°C per surface area of the needle shield in a buffer at pH 6.0, containing 20 mM histidine, 150 mM arginine, aspartic acid (q.s.), and 0.5 mg/mL poloxamer 188 is 7 ng/mm$^2$/day or less, more preferably 0.88 ng/mm$^2$/day or less. For example, the zinc leaching amount per surface area of the needle shield ($M_{zinc}(\mu g/mm^2)$) is determined from the following expression:

[Expression 1]

$$M_{zinc}(\mu g/mm^2) = (C_{sample} - C_{control}) \text{ (ppm) x } 10^{-3} \text{ x } 10 \text{ (mL/needle shield)}/700 \text{ (mm}^2\text{/needle shield)}$$

Here, $C_{sample}$ (ppm), $C_{control}$ (ppm), 10 (mL/needle shield), and 700 (mm$^2$/needle shield) are zinc concentration in zinc extraction solution, zinc concentration in control buffer, volume of extraction solution per needle shield, and approximate surface area per needle shield after being cut into four, respectively (see Example 2). The zinc leaching rate at 5°C per surface area of the needle shield ($k_{ext}(\mu g/mm^2/day)$) is calculated, for example, by determining the zinc leaching amount per surface area of the needle shield when extracted at 5°C for 3 days, 7 days, and 14 days, and calculating the rate from the slope (see Example 2).

In the present disclosure, the zinc leaching capacity can be tested for materials that meet a predetermined level as a container member for pharmaceutical products, and materials that are evaluated as having a low zinc leaching capacity can be selected and used.

In one aspect, an evaluation of low zinc leaching capacity can be performed by determining an expected zinc concentration in the needle to a predetermined buffer for an antibody solution formulation. For example, a needle shield material expected to have 5 mg/mL or less zinc concentration ($C_{zinc}(mg/mL)$) after 2 years at 5°C in the needle bevel under conditions of 20 mM histidine, 150 mM arginine, aspartic acid (q.s.), 0.5 mg/mL poloxamer 188, pH 6.0 can be selected and used. In one aspect, a needle shield material expected to have 0.63 mg/mL or less zinc concentration ($C_{zinc}(mg/mL)$) after 2 years at 5°C in the needle bevel under conditions of 20 mM histidine, 150 mM arginine, aspartic acid (q.s.), 0.5 mg/mL poloxamer 188, pH 6.0 can be selected and used.

For example, a zinc concentration ($C_{zinc}$(mg/mL)) after 2 years at 5°C in the needle bevel is determined by the expression below.

$$[\text{Expression 2}]$$

$$C_{zinc}(\text{mg/mL}) = k_{ext}(\mu g/mm^2/day) \times 730 \text{ (day)} \times 0.06 \text{ (mm}^2)/0.06 \text{ (}\mu L)$$

wherein, $k_{ext}$($\mu$g/mm$^2$/day), 0.06 (mm$^2$), and 0.06 ($\mu$L) are zinc leaching rate at 5°C per surface area of elastomer, cross-sectional area of needle bevel, and volume of drug solution in needle bevel, respectively (see Example 2).

In one embodiment, the material of the needle shield of the present disclosure is made of an elastomer. It is preferred that the elastomeric needle shield material has a low zinc leaching capacity to a buffer that can be used for an antibody formulation.

Elastomer

The elastomer is a generic term for an industrial material having a rubber elasticity (property of large deforming with a small force and reversing immediately when the force is removed), and the term is derived from the terms "elastic" and "polymer". The elastomer is classified into two groups: thermosetting elastomers that do not soften in a certain range even when heat is added, and have relatively high heat resistance; and thermoplastic elastomers that soften when heat is applied to exhibit fluidity, and have the property of returning to a rubbery state when cooled.

The thermosetting elastomers undergo a crosslinking reaction between polymer backbones when heated, and change their molecular structure into a three-dimensional mesh structure. There are various types of cross-linking reactions including: condensation reactions that release some by-product molecules when molecules are bound to each other, i.e., cross-linked; addition reactions that release nothing when the molecules are bound to each other; and reactions in which cross-linking occurs by irradiation with ultraviolet ray (UV) or electron beam (EB).

Examples of the thermosetting elastomer include silicone rubber, fluoro rubber, and urethane rubber (some of which may be classified as a thermoplastic elastomer). As an example, a silicone rubber contains a base polymer, a crosslinking agent, and a filler, and when an additive to promote a crosslinking reaction, such as a catalyst or a vulcanizing agent, is added thereto, the silicone rubber is crosslinked by heating to yield an elastomer.

Specific examples of the thermosetting elastomer include Sumitomo P-101A (chlorobutyl type, manufactured by Sumitomo Rubber Industries, Ltd.).

The thermoplastic elastomer (TPE) does not have a cross-linking structure, and has a configuration in which the two types of hard segment component (cross-linkable) and soft segment component (elastic) are chemically bonded (block polymer type) or mixed (blend type).

Examples of the thermoplastic elastomer (TPE or TPR) include a styrene type (TPS, for short), an olefin/alkene type (TPO), a vinyl chloride type (TPVC), a urethane type (TPU), an ester type (TPEE or TPC, for short), and an amide type (TPAE).

Specific examples of the thermoplastic elastomer include Stelmi 8550 (manufactured by STELMI ITALIA SpA).

Further, Sumitomo P-101A (chlorobutyl type, manufactured by Sumitomo Rubber Industries, Ltd.), BD260 (manufactured by Becton Dickinson Company), and Stelmi 8550 (manufactured by STELMI ITALIA SpA) are commercially available.

One example of the needle shield material suitable for use in the present disclosure is a thermoplastic elastomer (TPE). The thermoplastic elastomer is an elastomer having the property of softening by heating to exhibit fluidity, and returning to a rubbery state by cooling. In particular, an ethylene propylene diene methylene type thermoplastic elastomer is preferred.

Another example of the elastomeric needle shield material, that is suitable for use in the present disclosure, is a halogenated butyl rubber (isobutylene-isoprene rubber (IIR)). The halogenated butyl rubber includes a brominated butyl rubber (BIIR) and a chlorinated butyl rubber (CIIR). In particular, a chlorinated butyl rubber (chlorobutyl type elastomer) is preferred.

The structure of the needle cap is not particularly limited as long as the above-described material is formed in a tubular shape and the cap has an opening with one end closed and the other end attachable to surround and adhere to the injection needle or the outer periphery of the syringe body. The structure may be any of a single-layer, a multi-layer, or another structure. A groove or protrusion for installing or uninstalling the needle cap and/or needle shield may be provided inside the opening. The needle cap and/or needle shield may be attached to cover from the tip of the needle to one end of the outer barrel, the syringe body, or may be attached to cover from the tip of the needle to a connector portion of the injection needle and the syringe body.

Syringe body and Needle

The material of the syringe body of the syringe of the present disclosure is not particularly limited, and may be any material that can be normally used for a syringe. Specifically, the material may be glass, a resin, or the like.

When the prefilled syringe of the present disclosure is sterilized with radiation such as gamma rays, the material of the syringe body is preferably a material that is hardly affected by radiation (for example, hardly colored or deteriorated). Specific examples of the material include a resin such as a cycloolefin type resin, a polyethylene type resin, and a polypropylene type resin, and particularly preferably, a cycloolefin type resin such as a cyclic olefin polymer (COP) or cyclic olefin copolymer (COC). Such a syringe is particularly suitable for use as a syringe for tray filler filling which is used in mass production on an industrial scale.

The volume size (standard) of the syringe of the present disclosure is not particularly limited. Specifically, however, the advantageous effect of the present disclosure is remarkable when the volume of the syringe is small, such as 0.5 mL to 5.0 mL, preferably 1 mL.

The size of the injection needle is not particularly limited. Specifically, it is preferred that the outer diameter is 0.2 to 0.5 mm, and the inner diameter is 0.1 to 0.3 mm. Typical gauges of the injection needle are 25 (outer diameter 0.50-0.53 mm), 26 (outer diameter 0.44-0.47 mm), 27 (outer diameter 0.40-0.42 mm), 28 (outer diameter 0.34-0.37 mm), 29 (outer diameter 0.32-0.35 mm), 30 (outer diameter 0.29-0.32 mm), 31 (outer diameter 0.25-0.27 mm), 32 (outer diameter 0.22-0.24 mm), or 33G (outer diameter 0.20-0.22 mm), but the present disclosure is not limited to these sizes.

The prefilled syringe formulations of the present disclosure are typically self-injectable formulations. According to such configurations, a user of the prefilled syringe formulation is not a medical practitioner, but the patient himself. Even if the prefilled syringe formulation is improperly left for a long period, the risk of needle clogging can be reduced with the prefilled syringe formulation of the present disclosure.

Production of staked-in needle prefilled syringe formulation

Next, as another embodiment of the present disclosure, the production of a staked-in needle prefilled syringe formulation having a needle cap is described.

In a production of a staked-in needle prefilled syringe formulation having a needle cap, the syringe is generally filled with a drug solution using a tray filler, and thus the staked-in needle syringe is sterilized before filled with the drug solution, for example, by a syringe manufacturer. An assembly of a staked-in needle syringe is performed by attaching a injection needle to a tip of a syringe body and then attaching a needle cap to cover the injection needle. The staked-in needle syringe with the needle cap fitted is then sterilized by irradiating with radiation or electron beam for a time sufficient to sterilize. The most common radiation sterilization is performed by gamma ray irradiation and uses cobalt 60 or the like as a source of radiation, but is not limited to these. Since the gamma rays have excellent transmission power, there are no packaging form restrictions, the dose varies less, and the needle can be sterilized even fitted with the needle cap including an elastomeric needle shield having a low zinc leaching capacity. The dose of radiation depends on the amount of object to be sterilized, and typically about 10 kGy to 60 kGy, preferably about 25 kGy to 50 kGy are irradiated as absorbed doses of gamma radiation. After sterilization, the syringe body is filled with a drug solution in a sterile environment and then a plunger stopper is attached thereto. The staked-in needle prefilled syringe formulations may then be pillow-packaged in small packaging units, e.g., per syringe.

Antibody Formulations

The staked-in needle prefilled syringe formulation of the present disclosure is particularly preferably applied to high-concentration antibody-containing solution formulations, such as for subcutaneous injection.

In the present disclosure, the antibody-containing solution formulation refers to a solution formulation that contains an antibody as an active ingredient and is prepared so that it can be administered to an animal such as a human, and preferably produced without a lyophilization step in the production process.

One aspect of the present disclosure is a subcutaneous injection formulation for self-injection, in which a staked-in needle prefilled syringe is filled with a high-concentration antibody-containing solution formulation, and the needle cap described above is removably attached to the syringe body described above.

The high-concentration antibody-containing solution formulation of the present disclosure has an antibody concentration of 50 mg/mL or more, preferably 80 mg/mL or more, further preferably 100 mg/mL or more, furthermore preferably 120 mg/mL or more, and still more preferably 150 mg/mL or more.

The upper limit of the antibody concentration of the antibody-containing solution formulation of the present disclosure is generally 300 mg/mL, preferably 250 mg/mL, further preferably 200 mg/mL. Thus, the antibody concentration of the high-concentration antibody solution formulation of the present disclosure is preferably 50 to 300 mg/mL, further preferably 100 to 300 mg/mL, further preferably 120 to 250 mg/mL, and particularly preferably 150 to 200 mg/mL.

Antibody

In the present disclosure, the term "antibody" is used in the broadest sense, and includes monoclonal antibodies from animals such as humans, mice, rats, hamsters, rabbits, sheep, camels, and monkeys, as well as chimeric antibodies, humanized antibodies, small antibodies (including antibody fragments), multi-specific antibodies and the like as long as they exhibit the desired biological activity. In the present disclosure, these antibodies can be suitably obtained (produced) using the antibody engineering method of the present disclosure.

The present disclosure exhibits a remarkable effect in antibodies to which SMART technology has been applied. This is because such antibodies have a higher risk of gelation with zinc than antibodies not engineered with SMART technology.

SMART technology is a technology for introducing a property of antigen-binding activity weaker at pH in an early endosome than that at pH in plasma (blood) into a polypeptide having an antigen-binding ability, such as an antigen-binding molecule. Examples thereof include a recycling antibody. More specifically, it is characterized by the substitution of at least one amino acid with histidine or the insertion of at least one histidine in an antigen-binding molecule. SMART technology improves the pharmacokinetics of an antigen-binding molecule by enabling one molecule of the antigen-binding molecule to bind to a plurality of antigens repeatedly, or by binding one molecule of the antigen-binding molecule to a plurality of antigens (WO 2009125825, US 20110111406).

In the present disclosure, the position at which a histidine mutation (substitution or insertion) is introduced into the antibody is not particularly limited, and any site may be used as long as the antigen-binding activity at pH 5.8 becomes lower than the antigen-binding activity at pH 7.4 (the value of KD (pH 5.8)/KD (pH 7.4) becomes large) compared to before the mutation or insertion. When the antigen-binding molecule is an antibody, examples of the site include a variable region, preferably a CDR region, of the antibody. The number of histidine mutations or insertions introduced (performed) can be appropriately determined by a person skilled in the art, and may be substituted with histidine at one position only, or may be inserted with histidine at one position only, or may be substituted with histidine at two or more positions, or may be inserted with histidine at two or more positions. Alternatively, a mutation other than histidine mutation, such as a mutation with an amino acid other than histidine, may be introduced simultaneously. Furthermore, a histidine mutation and a histidine insertion may be performed simultaneously.

The SMART technology-applied antibody used in the present disclosure binds to a desired antigen with higher binding activity at neutral pH than at acidic pH. The phrase "binds to a desired antigen with higher binding activity at neutral pH than at acidic pH" means that the antigen-binding activity of the antigen-binding molecule at pH 4.0 to pH 6.5 is made lower than the antigen-binding activity at pH 6.7 to pH 10.0. Preferably, it means that the antigen-binding activity at pH of 5.5 to 6.5 of the antigen-binding molecule is made lower than the antigen-binding activity at pH of 7.0 to 8.0, and particularly preferably that the antigen-binding activity at pH 5.8 of the antigen-binding molecule is made lower than the antigen-binding activity at pH 7.4. Accordingly, the acidic pH in the present disclosure is typically pH 4.0 to pH 6.5, preferably pH 5.5 to pH 6.5, particularly preferably pH 5.8. The neutral pH in the present disclosure is typically pH 6.7 to pH 10.0, preferably pH 7.0 to pH 8.0, particularly preferably pH 7.4.

The phrase "antigen-binding ability of an antigen-binding molecule at acidic pH is made lower than antigen-binding ability at neutral pH" can also be expressed as the antigen-binding ability of the antigen-binding molecule at neutral pH is made higher than the antigen-binding ability at acidic pH. In other words, the difference between the antigen-binding ability at acidic pH and the antigen-binding ability at neutral pH of the antigen-binding molecule may be increased in the present disclosure. For example, the value of KD (pH 5.8)/KD (pH 7.4) may be increased as described later. To increase the difference between the antigen-binding ability of the antigen-binding molecule at acidic pH and the antigen-binding ability at neutral pH, for example, the antigen-binding ability at acidic pH may be reduced, or the antigen-binding ability at neutral pH may be increased, or both.

In one aspect, an antibody of the present disclosure has a value of KD (pH 5.8)/KD (pH 7.4), a ratio of KD at pH 5.8 to KD at pH 7.4 for an antigen, of 2 or more.

In one aspect, an antibody of the present disclosure has a value of KD (pH 5.8)/KD (pH 7.4) of 10 or more.

In one aspect, an antibody of the present disclosure has a value of KD (pH 5.8)/KD (pH 7.4) of 40 or more.

In one aspect, an antibody of the present disclosure is an antibody characterized by having an antagonist activity.

In one aspect, an antibody of the present disclosure is an antibody characterized by binding to a membrane antigen or a soluble antigen.

In one aspect, an antibody of the present disclosure is characterized in that at least one amino acid is substituted with histidine.

In one aspect, an antibody of the present disclosure is characterized in that at least one histidine is inserted.

In one aspect, an antibody of the present disclosure has a substitution of at least one amino acid with histidine or an insertion of at least one histidine.

An antibody of the present disclosure may be a polyclonal antibody or a monoclonal antibody, but a monoclonal antibody is preferred in that a homogeneous antibody can be stably produced.

KD (dissociation constant) can be used as the value of antigen-binding activity when the antigen is a soluble antigen, while apparent KD (apparent dissociation constant) can be used when the antigen is a membrane antigen. KD (dissociation constant), and apparent KD (apparent dissociation constant) can be measured by methods known to those skilled in the art, for example, using Biacore (GE healthcare), a Scatchard plot, FACS, or the like.

In the present disclosure, as another indicator showing the difference between antigen-binding activity at acidic pH and antigen-binding activity at neutral pH, for example, k d (dissociation rate constant), which is a dissociation rate constant, can also be used. When k d (dissociation rate constant) is used instead of KD (dissociation rate constant) as an indicator showing the difference in binding activity, the value of k d (pH 5.8)/k d (pH 7.4), which is the ratio of k d (dissociation rate constant) at pH 5.8 to k d (dissociation rate constant) at pH 7.4 for an antigen, is preferably 2 or more, further preferably 5 or more, furthermore preferably 10 or more, still more preferably 30 or more. The upper limit of the value of k d (pH 5.8)/k d (pH 7.4) is not particularly limited, and may be any value, such as 50, 100, or 200, as far as it can be made with the ordinary skill of a skilled person.

k d (dissociation rate constant) can be used as the value of antigen-binding activity when the antigen is a soluble antigen, but an apparent k d (apparent dissociation rate constant) can be used when the antigen is a membrane antigen. k d (dissociation rate constant) and apparent k d (apparent dissociation rate constant) can be measured by methods known to those skilled in the art, for example, using Biacore (GE healthcare), FACS, or the like.

Note that, in the present disclosure, it is preferred that the conditions other than pH be the same when the antigen-binding activities of an antigen-binding molecule at a different pH are measured.

The term "antigen" in the present disclosure refers to a molecule that initiates an immune reaction. This immune reaction may include either antibody production or activation of specific immunocompetent cells, or both. Every high molecule including substantially all proteins or peptides may be useful as the antigen. One aspect of the antigen can include a receptor protein (such as a membrane-bound receptor, a soluble receptor), a membrane antigen such as a cell surface marker, and a soluble antigen such as a cytokine. Preferred examples of the membrane antigen in the present disclosure can include a membrane protein. Examples of the soluble antigen in the present disclosure can include a soluble protein. Specific examples of the antigen can include IL-1, IL-2, IL-3, IL-4, IL-5, IL-6, IL-7, IL-8, IL-9, IL-10, IL-11, IL-12, IL-15, IL-31, IL-23, an IL-2 receptor, an IL-6 receptor, an OSM receptor, gp130, an IL-5 receptor, CD40, CD4, Fas, osteopontin, myostatin, CRTH2, CD26, PDGF-D, CD20, a monocyte chemotactic factor, CD23, TNF-$\alpha$, HMGB-1, $\alpha$4 integrin, ICAM-1, CCR2, CD11a, CD3, IPN$\gamma$, BLyS, HLA-DR, TGF-$\beta$, CD52, and an IL-31 receptor. Particularly preferred antigens include an IL-6 receptor, IL-8, and/or myostatin.

The present disclosure also exhibits a remarkable effect in antibodies to which pI engineering technology has been applied in addition to SMART technology. This is because such antibodies have a higher risk of gelation with zinc than antibodies to which SMART technology and pI engineering technology have not been applied.

The pI engineering technology used in the present disclosure includes a lowering pI technology in which a charge of at least one amino acid residue that can be exposed to a surface of the CDR region is engineered. More specifically, amino acid residues at positions 31, 62, 64, and 65 by Kabat numbering in the heavy chain variable region or amino acid residues at positions 24, 27, 53, 54, and 55 by Kabat numbering in the light chain variable region that can be exposed to a surface of the CDR region are substituted with amino acid residues selected from amino acid residues included in either group of: (a) glutamic acid (E) and aspartic acid (D); or (b) lysine (K), arginine (R), and histidine (H). The plasma half-life of a polypeptide containing a variable region of an antibody can be suppressed by controlling the isoelectric point of the polypeptide (WO 2009041643, US 20100239577 A1). Whether or not the pI engineering technology is applied can be evaluated by the change of the isoelectric point in the comparison between the antibodies before and after engineering, as when antibodies are added to an electrophoretic gel and subjected to an electric field, each antibody moves toward a pH that is same as its natural pI through the electrophoretic gel in which a pH gradient is formed.

In the present disclosure, the phrase "antibody has one or more histidine residue pairs that are adjacent to each other within three residues in a CDR region" means that two histidine residues are arranged in succession (histidine-histidine) or are arranged with one amino acid interposed therein (histidine-amino acid-histidine) in CDR 1, 2, and/or 3 regions. In one aspect, the antibody may have at least one histidine residue pair. The antibody may have one or more histidine residue pairs that are adjacent to each other within three residues in a CDR region in a sequence resulting from an antibody to which SMART technology and/or pI engineering technology have been applied.

In the present disclosure, the phrase "antibody has a total number of histidine, aspartic acid, and glutamic acid present in a CDR region of 6 or more" means that 6 or more three amino acids described above in total are contained in CDR 1, 2, and/or 3 regions regardless of the position or arrangement. In one aspect, the total number of the three amino acids is 6 or more, 7 or more, or 12 or more. The antibody may have a total number of histidine, aspartic acid, and glutamic acid present in a CDR region of 6 or more, 7 or more, or 12 or more in a sequence resulting from an antibody to which SMART technology and/or pI engineering technology have been applied.

The "antibody" in the present disclosure includes an antibody in which an amino acid sequence of the antibody is further engineered by applying amino acid substitution, deletion, addition, and/or insertion, or the like to an antibody in which a charge of an amino acid residue has been engineered as described above. The "antibody" also includes an antibody in which a charge of an amino acid residue of the antibody is further engineered by applying the change to an antibody in which an amino acid sequence has been engineered by amino acid substitution, deletion, addition, and/or insertion, chimerization, humanization, or the like. That is, the mouse antibody may be engineered simultaneously in a humanizing step, or the humanized antibody may be further engineered. One aspect includes a case in which, when a chimeric antibody is humanized, the pI of the chimeric antibody is engineered by engineering an amino acid residue that can be exposed to a surface of the chimeric antibody. Another aspect includes a case in which the pI of the human antibody is engineered by engineering an amino acid residue that can be exposed to a surface of a human antibody produced from a human antibody library, a human antibody-producing mouse, or the like.

The immunoglobulin class of the antibody used in the present disclosure is not particularly limited, and any of the class of IgG such as IgG1, IgG2, IgG3, or IgG4, IgA, IgD, IgE, IgM, or the like may be used, but IgG and IgM are preferred.

Furthermore, the antibody used in the present disclosure includes not only an antibody having a constant region and a variable region (i.e., whole antibody) but also low molecular weight antibodies. The low molecular weight antibodies include antibody fragments such as Fv, Fab, F(ab)$_2$, monovalent or di- or multi-valent single-chain Fv (scFv, sc(Fv)$_2$) and a diabody such as an scFv dimer in which variable regions of the antibody is attached by a linker such as a peptide linker, and the like. However, the whole antibody is preferred as the antibody used in the present disclosure.

The antibody used in the present disclosure as described above can be produced by methods well known to those skilled in the art. Monoclonal antibody-producing hybridomas can be basically produced using known techniques as follows. That is, a desired antigen or a cell expressing the desired antigen is used as a sensitizing antigen to immunize cells according to a normal immunization method. The resulting immunized cells are fused with known parental cells by a normal cell fusion method. Then, monoclonal antibody-producing cells (hybridomas) are screened by a normal screening method to produce the hybridomas. Production of hybridomas can be performed, for example, according to methods of Miller et al. (Kohler. G. and Milstein, C., Methods Enzymol. (1981) 73: 3-46), and the like. When the immunogenicity of the antigen is low, the antigen may be conjugated to a macromolecule having immunogenicity, such as albumin, to immunize.

Alternatively, a recombinant antibody can be used, which is produced using a genetic recombination technology by cloning an antibody gene from a hybridoma, incorporating it into an appropriate vector, and introducing it into a host (see, e.g., Carl, A. K. Borrebaeck, James, W. Larrick, therapeutic monoclonal antibodies, published in the United Kingdom by Macmillan publishers Ltd., 1990). Specifically, cDNA of a variable region (V region) of an antibody is synthesized from mRNA of the hybridoma using a reverse transcriptase. Once a DNA encoding the V region of an antibody of interest is obtained, it is linked to a DNA encoding a constant region (C region) of a desired antibody, and incorporated into an expression vector. Alternatively, the DNA encoding the V region of the antibody may be incorporated into an expression vector containing a DNA of an antibody C region. The DNA is incorporated into the expression vector to express under the control of an expression control region, e.g., an enhancer or a promoter. This expression vector can be used to transform a host cell to express an antibody.

In the present disclosure, a gene recombinant antibody that is artificially engineered to decrease the heterologous antigenicity against humans, such as a chimeric antibody or a humanized antibody, can be used. These engineered antibodies can be produced using known methods. The chimeric antibody is an antibody consisting of a variable region of a heavy chain and a light chain of a non-human mammal antibody, such as a mouse antibody, and a constant region of a heavy chain and a light chain of a human antibody. A chimeric antibody can be obtained by linking a DNA encoding a variable region of a mouse antibody to a DNA encoding the constant region of a human antibody, integrating the resultant to an expression vector, and introducing the obtained vector into a host to produce a chimeric antibody.

A humanized antibody, also referred to as a reshaped human antibody, is an antibody in which a CDR region of a non-human mammal antibody, such as a mouse antibody, is implanted into a CDR region of a human antibody. A typical genetic recombination method of a humanized antibody is also known. Specifically, a DNA sequence designed to ligate a CDR region of a mouse antibody to a framework region (FR) of a human antibody is synthesized by PCR from a number of oligonucleotides prepared to have overlapping portions at the ends. The resulting DNA is linked to a DNA encoding a human antibody constant region, then incorporated into an expression vector, and introduced into a host to produce a humanized antibody. (See, European Patent Application Publication No. EP 239 400, and WO 96/02576). The FR region of the human antibody that is linked via the CDR region is selected so that the CDR region forms a good antigen-binding site. If needed, amino acids of the FR region of the variable region of the antibody may be substituted such that the CDR region of the reconstituted human antibody forms an appropriate antigen-binding site (Sato, K. et al., Cancer Res. (1993) 53, 851-856).

Techniques for substituting an amino acid of an antibody to improve the activity, physical properties, pharmacokinetics, safety, and the like of the antibody are also known, such as the techniques described below. Antibodies in which such amino acid substitutions (including deletions and additions) have been applied are also included in the antibody used in the present disclosure.

As techniques for applying amino acid substitutions on a variable region of an IgG antibody, humanization (Tsurushita N, Hinton PR, Kumar S., Design of Humanized Antibodies: from anti-Tac to Zenapax., Methods. 2005 May; 36 (1): 69-83.), affinity maturation by amino acid substitution of the CDR region to enhance binding activity (Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc Natl Acad Sci USA. 2005 Jun 14; 102 (24): 8466-71.), and improvements in the physicochemical stability by amino acid substitutions in the framework (FR) (Ewert S, Honegger A, Pluckthun A., Stability improvement of antibodies for extracellular and intracellular applications: CDR grafting to stable frameworks and structure-based framework engineering., Methods. 2004 Oct. 34 (2): 184-99, Review) have been reported. Also, as techniques for performing amino acid substitutions in the Fc region of IgG antibodies, techniques for enhancing antibody-dependent cytotoxic activity (ADCC) activity and complement-dependent cytotoxic activity (CDC) activity are known (Kim SJ, Park Y, Hong HJ., Antibody engineering for the development of the therapeutic antibodies., Mol Cells. 2005 Aug 31; 20 (1): 17-29. Review.). Furthermore, techniques for amino acid substitution of Fc that not only enhance such effector functions but also improve the blood half-life of the antibody have been reported (Hinton PR, Xiong JM, Johlfs MG, Tang MT, Keller S, Tsurushita N., An engineered human IgG1 antibody with longer serum half-life., J Immunol. 2006 Jan 1;176(1):346-56, Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul; 15(7):637-40.). Various amino acid substitution techniques for constant regions aimed at improving the physical properties of antibodies are also known (WO 09/41613).

Methods for obtaining human antibodies are also known. For example, a desired human antibody having a binding activity to an antigen can be obtained by sensitizing human lymphocytes in vitro with a desired antigen or a cell expressing the desired antigen, and fusing the sensitized lymphocytes with human myeloma cells, e.g., U266 (see, JP 1-59878 B). The desired human antibody can also be obtained by immunizing a transgenic animal with an antigen having all the repertoires of the human antibody gene (see WO 93/12227, WO 92/03918, WO 94/02602, WO 94/25585, WO 96/34096, WO 96/33735). Furthermore, a technique of obtaining a human antibody by panning using a human antibody library is also known. For example, it is possible to select a phage that binds to an antigen by expressing a variable region of a human antibody as a single-chain antibody (scFv), on the surface of the phage, by a phage display method. By analyzing the gene of the selected phage, it is possible to determine the DNA sequence encoding a variable region of a human antibody that binds to the antigen. Once the DNA sequence of the scFv that binds to the antigen is revealed, an appropriate expression vector containing the sequence can be generated and a human antibody can be obtained. These methods are already well known and can be referred to WO 92/01047, WO 92/20791, WO 93/06213, WO 93/11236, WO 93/19172, WO 95/01438, WO 95/15388. The antibody used in the present disclosure also includes such human antibodies.

When an antibody gene is isolated once and introduced into an appropriate host to produce an antibody, an appropriate combination of a host and an expression vector can be used. When a eukaryotic cell is used as the host, an animal cell, a plant cell, and a fungal cell can be used. As the animal cell, (1) a mammalian cell, such as CHO, COS, myeloma, BHK (baby hamster kidney), HeLa, Vero, (2) an amphibian cell, such as Xenopus oocyte, and (3) an insect cell, such as sf9, sf21, or Tn5, are known. As the plant cell, a cell from the genus Nicotiana, such as Nicotiana tabacum, is known and may be cultivated by callus. As the fungal cell, a yeast, for example, of the genus Saccharomyces, for example, Saccharomyces serevisiae, filamentous fungi, for example, of the genus Aspergillus, such as Aspergillus niger, are known. When a prokaryotic cell is used, a production system using a bacterial cell can be used. E. coli and B. subtilis are known as the bacterial cell. An antibody gene of interest is introduced into these cells by transformation, and the transformed cells are cultured in vitro to yield an antibody.

The antibody used in the present disclosure further includes a modified antibody. For example, antibodies linked to various molecules, such as polyethylene glycol (PEG) and cytotoxic agents, can also be used (Farmaco. 1999 Aug 30; 54 (8): 497-516., Cancer J. 2008 May-Jun; 14 (3): 154-69.). The antibody used in the present disclosure also encompasses such a modified antibody. Such a modified antibody can be obtained by chemically modifying the antibody. These methods have already been established in this field.

Examples of the antibody used in the present disclosure include, but are not limited to, an anti-IL-6 receptor antibody, an anti-IL-6 antibody, an anti-IL-8 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, anti-CD137 (4-1BB) antibody, an anti-C5 antibody, an anti-Ang-2 antibody, an anti-myostatin antibody, an anti-IL-31 receptor antibody, an anti-AXL antibody, an anti-CXCR4 antibody, an anti-CTLA-4 antibody, a bi-specific antibody of Factor IX and Factor X, an anti-PD-L1 antibody, an anti-CEA antibody, an anti-amyloid beta antibody, anti-$\alpha$-synuclein antibody, an anti-VAP-1 antibody, an anti-RANKL antibody, an anti-BLyS antibody, and an anti-CCR4 antibody.

Preferred examples of the reconstituted humanized antibody used in the present disclosure include an anti-IL-6 receptor antibody, an anti-IL-6 antibody, an anti-IL-8 antibody, an anti-glypican-3 antibody, an anti-CD3 antibody, an anti-CD20 antibody, an anti-Her2/neu antibody, an anti-4-1BB (CD137) antibody, an anti-C5 antibody, an anti-VEGF antibody, an anti-Ang-2 antibody, an anti-myostatin antibody, an anti-IL-31 receptor antibody, a Bi-specific antibody between Factor IX and Factor X, an anti-PD-L1 antibody, an anti-CEA antibody, an anti-amyloid beta antibody, and an anti-α-synuclein antibody, and more preferred antibodies include an anti-IL-6 receptor antibody, an anti-IL-8 antibody, an anti-myostatin antibody, and anti-IL-31 receptor antibody.

Particularly preferred examples of the reconstituted humanized antibody used in the present disclosure are a humanized anti-IL-6 receptor antibody (satralizumab (RG6168)), an anti-IL-8 antibody (mAb2 described herein), and an anti-myostatin antibody (RG6237).

mAb2 is an anti-IL-8 antibody as described in JP 6266164 B, and identified as described in A, B, or C below.

A. An anti-IL-8 antibody including: (a) HVR-H1 including the amino acid sequence of SEQ ID NO: 67; (b) HVR-H2 including the amino acid sequence of SEQ ID NO: 73; (c) HVR-H3 including the amino acid sequence of SEQ ID NO: 74; (d) HVR-L1 including the amino acid sequence of SEQ ID NO: 70; (e) HVR-L2 including the amino acid sequence of SEQ ID NO: 75; and (f) HVR-L3 including the amino acid sequence of SEQ ID NO: 76.

B. An anti-IL-8 antibody including a heavy chain variable region of SEQ ID NO: 78 and a light chain variable region of SEQ ID NO: 79.

C. An anti-IL-8 antibody including a heavy chain including the amino acid sequence of SEQ ID NO: 80 or SEQ ID NO: 81 and a light chain including the amino acid sequence of SEQ ID NO: 82.

The "antibody" in the present disclosure includes a biosimilar of the antibody. The "biosimilar" in the present disclosure refers to a biological agent that may be an antibody having the same primary amino acid sequence as a commercially available antibody (also referred to as a "product of interest") but can be produced in different cells or by different methods of manufacture, purification or formulation, and is similar to the product of interest based on data derived from (i) analytical studies demonstrating that the antibody formulation is highly similar to the product of interest, (ii) animal studies (including assessment of toxicity), and/or (iii) one or a plurality of clinical studies (including assessments of immunogenicity and pharmacokinetics or pharmacodynamics) in which the product of interest is approved for use, intended for use, and sufficient to demonstrate safety, purity, and potency under one or more suitable conditions of use for the formulation.

In the present disclosure, an antibody having a low isoelectric point (low pI antibody) refers to an antibody having a low isoelectric point that is difficult to occur, particularly, in nature. The isoelectric points of such antibodies include, but are not limited to, 3.0 to 8.0, preferably 5.0 to 7.5, further preferably 5.0 to 7.0, and particularly preferably 5.0 to 6.5. It should be noted that antibodies in nature (or conventional antibodies) are generally believed to have isoelectric points ranging from 7.5 to 9.5.

Furthermore, the antibody used in the present disclosure is preferably a pI engineered antibody in which the pI of the antibody is reduced by engineering an amino acid residue that can be exposed to a surface of the antibody. Such a pI-engineered antibody refers to an antibody in which the pI is reduced by 1 or more, preferably 2 or more, and further preferably 3 or more, compared tothan the pI of the antibody before the engineering. Examples of such a pI-engineered antibody include, but are not limited to, satralizumab (SA237, or MAb3 herein), which is an anti-IL-6 receptor antibody described in WO 2009/041621, and an anti-NR10 humanized antibody, which is a fully-humanized NS22 antibody produced by the method described in Example 12 of WO 2009/072604.

For an H-chain variable region, examples of the amino acid residue that can be exposed to a surface of the antibody include, but are not limited to, an amino acid residue selected from H1, H3, H5, H8, H10, H12, H13, H15, H16, H19, H23, H25, H26, H31, H39, H42, H43, H44, H46, H61, H62, H64, H65, H68, H71, H72, H73, H75, H76, H81, H82b, H83, H85, H86, H105, H108, H110, and H112, which are amino acid residues based on Kabat numbering. For an L-chain variable region, examples thereof include, but are not limited to, an amino acid residue selected from L1, L3, L7, L8, L9, L11, L12, L16, L17, L18, L20, L22, L24, L27, L38, L39, L41, L42, L43, L45, L46, L49, L53, L54, L55, L57, L60, L63, L65, L66, L68, L69, L70, L74, L76, L77, L79, L80, L81, L85, L100, L103, L105, L106, and L107, which are amino acid residues based on Kabat numbering.

The "engineered" in the present disclosure refers to substituting an original amino acid residue with another amino acid residue, deleting the original amino acid residue, adding a new amino acid residue, and the like, but preferably, substituting the original amino acid residue with another amino acid residue.

In amino acids, it is known that there are some charged amino acids. In general, lysine (K), arginine (R), and histidine (H) are known as positively charged amino acids (positive-charge amino acids). As negatively charged amino acids (negative-charge amino acids), aspartic acid (D), glutamic acid (E), and the like are known. Amino acids other than these are known as uncharged amino acids.

The amino acid residue that has been engineered in the present disclosure is preferably, but is not particularly limited to, selected suitably from the amino acid residues included in any of the following groups (a) or (b):

(a) glutamic acid (E) and aspartic acid (D);
(b) lysine (K), arginine (R), and histidine (H).

When the amino acid residue before the engineering has already had a charge, one preferred aspect is engineering the amino acid residue to an amino acid residue that does not have a charge.

That is, the engineering in the present disclosure includes: (1) a substitution from an amino acid having a charge to an amino acid not having a charge; (2) a substitution from an amino acid having a charge to an amino acid having a charge opposite to the charge; and (3) a substitution from an amino acid not having a charge to an amino acid having a charge.

The value of the isoelectric point can be measured by isoelectric electrophoresis known to those skilled in the art. The value of the theoretical isoelectric point can be calculated using gene and amino acid sequence analysis software (such as Genetyx).

An antibody in which a charge of the amino acid residue has been engineered can be obtained by engineering a nucleic acid encoding the antibody, culturing the nucleic acid in a host cell, and purifying the antibody from a culture of the host cell. As used in the present disclosure, the phrase "engineering a nucleic acid" refers to engineering a nucleic acid sequence to have a codon corresponding to an amino acid residue to be introduced by the engineering. More specifically, it refers to engineering a nucleotide sequence of a nucleic acid such that a codon of an amino acid residue before the engineering becomes a codon of the amino acid residue to be introduced by the engineering. That is, a codon encoding the amino acid residue to be engineered is substituted with a codon encoding the amino acid residue to be introduced by the engineering. Such nucleic acid engineering can be performed as appropriate using techniques known to those skilled in the art, such as a site-directed mutagenesis method, a PCR mutagenesis method, or the like.

In the high concentration antibody-containing solution formulation used in the present disclosure, it is preferred that the antibody is an antibody in which at least one amino acid residue is substituted with histidine.

Buffered solution for antibody formulation

The buffer used in the protein-containing solution formulation of the present disclosure is prepared using a buffering agent that is a substance for maintaining the pH of the solution. In the high concentration antibody-containing solution formulation of the present disclosure, the pH of the solution is preferably 4 to 8, more preferably 5.0 to 7.5, further preferably 5.0 to 7.2, still more preferably 5.5 to 7.0, and still more preferably 5.8 to 6.2. The buffering agent that can be used in the present disclosure is a buffering agent that is pharmaceutically acceptable and can adjust the pH in this range. Such a buffering agent is known to those skilled in the art of solution formulations, and examples thereof include inorganic salts such as phosphate (sodium or potassium phosphate), sodium hydrogen carbonate, and the like; organic acid salts such as citrate (sodium or potassium citrate), sodium acetate, sodium succinate and the like; and acids such as phosphoric acid, carbonic acid, citric acid, succinic acid, malic acid, or gluconic acid. Further examples include Tris and Good buffering agents such as MES, MOPS, or HEPES, histidine (e.g., histidine hydrochloride), glycine, and mixtures of buffering agents (e.g., histidine-aspartic acid, and histidine-acetic acid).

In the high-concentration antibody-containing solution formulation of the present disclosure, the buffer is preferably a histidine buffer or a glycine buffer, particularly preferably a histidine buffer. The concentration of the buffer is generally 1 to 500 mM, preferably 5 to 100 mM, further preferably 10 to 20 mM. When a histidine buffer is used, the buffer preferably contains 5 to 25 mM histidine, further preferably 10 to 20 mM histidine.

The high concentration antibody-containing solution formulation of the present disclosure is preferably stabilized by adding a stabilizer suitable for the antibody that is an active ingredient. The "stable" high concentration antibody-containing solution formulation of the present disclosure has no significant change observed for at least 12 months, preferably two years, further preferably three years at refrigerated temperature (2-8°C); or for at least three months, preferably six months, further preferably one year at room temperature (22-28°C). For example, the total amount of the dimer and the decomposition product after 2 years of storage at 5°C is 5.0% or less, preferably 2% or less, further preferably 1.5% or less, or the total amount of the dimer and the decomposition product after 6 months of storage at 25°C is 5.0% or less, preferably 2% or less, further preferably 1.5% or less.

The formulations of the present disclosure can further contain a surfactant.

Examples of the surfactant include a nonionic surfactant such as a sorbitan fatty acid ester such as sorbitan monocaprylate, sorbitan monolaurate, or a sorbitan monopalmitate; a glycerol fatty acid ester such as glycerol monocaprylate, glycerol monomyristate, or glycerol monostearate; a polyglycerol fatty acid ester such as a decaglycerol monostearate, a decaglycerol distearate, or a decaglycerol monolinolate; a polyoxyethylene sorbitan fatty acid ester such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, or polyoxyethylene sorbitan tristearate; a polyoxyethylene sorbit fatty acid ester such as polyoxyethylene sorbit tetrastearate, or polyoxyethylene sorbit tetraoleate; a polyoxyethylene glycerine fatty acid ester such as polyoxyethylene glyceryl monostearate; a polyethylene glycol fatty acid ester such as polyethylene glycol distearate; a polyoxyethylene alkyl ether such as polyoxyethylene lauryl ether; a polyoxyethylene polyoxypropylene alkyl ether such as polyoxyethylene polyoxypropylene glycol ether, polyoxyethylene polyoxypropylene propyl ether, or polyoxyethylene polyoxypropylene cetyl ether; a polyoxyethylene alkylphenyl ether such as polyoxyethylene nonylphenyl ether; a polyoxyethylene hardened castor oil such as polyoxyethylene castor oil (polyoxyethylene hydrocarbon castor oil); a polyoxyethylene beewax derivative such as polyoxyethylene sorbit beewax; a polyoxyethylene lanolin derivative such as polyoxyethylene lanolin; those having HLB 6 to 18 such as polyoxyethylene fatty acid amide such as polyoxyethylene stearate amide; an anionic surfactant such as an alkyl sulfate having an alkyl group of 10 to 18 carbon atoms, such as sodium cetyl sulfate, sodium lauryl sulfate, or sodium oleyl sulfate; a polyoxyethylene alkyl ether sulfate having an average molar number of ethylene oxide of 2 to 4 and an alkyl group having 10 to 18 carbon atoms, such as sodium polyoxyethylene lauryl sulfate; an alkyl sulfosuccinate ester salt having 8 to 18 carbon atoms in the alkyl group, such as sodium lauryl sulfosuccinate ester; a naturally occurring surfactant such as lecithin, glycerophospholipid; a sphingophospholipid such as sphingomyelin; and a sucrose fatty acid ester of a fatty acid having 12 to 18 carbon atoms. One or two or more of these surfactants can be added to the formulations of the present disclosure in combination.

Preferred surfactants are polyoxyethylene sorbitan fatty acid esters and polyoxyethylene polyoxypropylene alkyl ethers, particularly preferred surfactants are polysorbates 20, 21, 40, 60, 65, 80, 81, and 85 and Pluronic (registered trademark) type surfactants, and most preferred surfactants are polysorbates 20, 80 and Pluronic F-68 (poloxamer 188; PX188).

The amount of the surfactant added to the antibody formulation of the present disclosure is generally 0.0001 to 10% (w/v), preferably 0.001 to 5%, further preferably 0.005 to 3%.

The formulation of the present disclosure can optionally contain a suspending agent, a dissolution aid, an isotonic agent, a preservative, an anti-adsorption agent, a diluent, an excipient, a pH adjuster, an analgesic agent, a sulfur-containing reducing agent, an antioxidant, and the like.

Examples of the suspending agent include methylcellulose, polysorbate 80, hydroxyethylcellulose, gum arabic, tragacanth powder, sodium carboxymethylcellulose, and polyoxyethylene sorbitan monolaurate.

Examples of the solution aid include polyoxyethylene cured castor oil, polysorbate 80, nicotinic acid amide, polyoxyethylene sorbitan monolaurate, macrogol, and castor oil fatty acid ethyl esters.

Examples of the isotonic agent include sodium chloride, potassium chloride, and calcium chloride.

Examples of the preservative include methyl paraoxybenzoate, ethyl paraoxybenzoate, sorbic acid, phenol, cresol, and chlorocreazole.

Examples of the anti-adsorption agent include human serum albumin, lecithin, dextran, ethylene oxide-propylene oxide copolymer, hydroxypropyl cellulose, methyl cellulose, polyoxyethylene cured castor oil, and polyethylene glycol.

Examples of the sulfur-containing reducing agent include N-acetylcysteine, N-acetylhomocysteine, thioctic acid, thiodiglycol, thioethanolamine, thioglycerol, thiosorbitol, thioglycol acid and a salt thereof, sodium thiosulfate, glutathione, methionine, and those having a sulfhydryl group such as a thioalkanoic acid having 1 to 7 carbon atoms.

Examples of the antioxidant include erythorbic acid, dibutyl hydroxytoluene, butyl hydroxyanisole, alpha-tocopherol, tocopherol acetate, L-ascorbic acid and a salt thereof, L-ascorbic acid palmitate, L-ascorbic acid stearate, sodium bisulfite, sodium sulfite, triamyl gallate, propyl gallate, or a chelating agent such as disodium ethylenediaminetetraacetate (EDTA), sodium pyrophosphate, or sodium metaphosphate.

Use

The antibody-containing prefilled syringe solution formulation of the present disclosure is administered subcutaneously, intravenously, intramuscularly, or the like. For subcutaneous injections, the dose of the antibody per injection is large, while the injection volume is limited. Thus, the formulation of the present disclosure is particularly suitable for subcutaneous injection.

The osmolality ratio of the antibody-containing solution formulation of the present disclosure is preferably about 0.5 to 4, more preferably about 0.7 to 2, and further preferably about 1. Here, the osmolality ratio is based on 309 mOsm/kg.

The viscosity of the antibody-containing solution formulation of the present disclosure is preferably about 2 to 100 mPa·s (pascal seconds), more preferably about 2 to 50 mPa·s, further preferably 2 to 30 mPa·s, further preferably about 4 to 50 mPa·s, furthermore preferably about 6 to 50 mPa·s. The viscosity in the present disclosure measured here is a viscosity measured at 25°C by an electromagnetic spinning method with an EMS viscometer described in Example 3, or a rotating viscometer method with a cone plate type viscometer (Japanese Pharmacopoeia 15th Edition, General Tests, 2.53 Viscosity Determination Method), and is a viscosity of the antibody-containing solution before filling the interior of the syringe body. Specifically, in the electromagnetic spinning method with an EMS viscometer, the viscosity is measured by placing an antibody solution (180 mg/mL) and an aluminum ball having a diameter of 2 mm in a glass tube having an inner diameter of 6.3 mm, and measuring the viscosity at 25°C with a rotational speed of 1000 rpm. The antibody solution is formulated with 20 mM histidine, 150 mM arginine, aspartic acid (q.s.), 0.5 mg/mL poloxamer 188, pH 6.0. To prevent adsorption of the sample to the aluminum ball, the aluminum ball is previously treated by a silicon coating in a desiccator with steam of dimethyldichlorosilane.

In one aspect, the antibody solution formulation of the present disclosure has a concentration of the antibody of 100 to 300 mg/ml and a viscosity of 6 to 100 mPa·s (pascal seconds), preferably, a concentration of the antibody of 100 to 300 mg/ml and a viscosity of 2 to 30 mPa·s (pascal seconds).

When the viscosity of the solution containing the protein is greater than 200 mPa·s (pascal seconds), preferably greater than 100 mPa·s, more preferably greater than about 50 mPa·s, and further preferably greater than 40 mPa·s, pushing out the drug from of the syringe becomes difficult, and the needle clogging is likely to occur within a short time (within 10 minutes) after removing the needle cap from the needle of the syringe. That is, when the viscosity of the protein-containing solution of the present disclosure is 6 to 100 mPa·s, preferably about 6 to 30 mPa·s, preferably 8 to 50 mPa·s, and further preferably about 20 to 50 mPa·s without zinc addition, the needle cap of the present disclosure is required.

Even when the protein concentrations are the same, the viscosity of the formulation may vary depending on components other than the antibody in the formulation. As the viscosity increases, the risk of needle clogging increases.

Examples of one aspect of the high-concentration antibody solution formulation enclosed by the prefilled syringe of the present disclosure include the solution formulations having the antibody concentration of 100 to 300 mg/mL and the viscosity of 6 to 100 mPa·s; the antibody concentration of 100 to 300 mg/mL, and the viscosity of 8 to 50 mPa·s; the antibody concentration of 100 to 300 mg/mL, and the viscosity of 20 to 50 mPa·s; the antibody concentration of 100 to 300 mg/mL, and the viscosity of 6 to 30 mPa·s; the antibody concentration of 120-250 mg/mL and viscosity of 6-100 mPa·s; the antibody concentration of 120-250 mg/mL and the viscosity of 8-50 mPa·s; the antibody concentration of 120-250 mg/mL and the viscosity of 20- 50 mPa·s; the antibody concentration 120-250 mg/mL, viscosity 6-30 mPa·s; the antibody concentration 150-200 mg/mL and the viscosity of 6-100 mPa·s; the antibody concentration 150-200 mg/mL and the viscosity of 8-100 mPa·s; the antibody concentration 150-200 mg/mL and the viscosity of 20-50 mPa·s; and the antibody concentration 150-200 mg/mL and the viscosity of 6-30 mPa·s. According to one aspect, the antibody concentration and the viscosity is those of an antibody solution in a formulation solution at pH 6.0, containing 20 mM histidine, 150 mM arginine, aspartic acid (q.s.), 0.5 mg/mL, and poloxamer 188 at the measured temperature of 25°C.

Examples

Hereinafter, the present disclosure will be described in more detail based on Examples, but the scope of the present disclosure is not limited to these.

EXAMPLE 1

Example 1: Zinc leaching capacity from elastomer in harsh conditions

The zinc leaching capacity from the elastomer of the needle shield was evaluated. As the elastomer, five elastomers commonly used for needle shields were used (Table 1). Each elastomer was cut into two in the longitudinal direction and used. In a pre-washed glass bottle with a ground-in stopper, 10 g of cut elastomer, 20 mL of water (neutral), 10 mM phosphoric acid solution (acidic), or 10 mM sodium hydroxide solution (alkaline) were added and closed with the stopper, then the stopper was fixed with a wire clamp, and the bottle was incubated at 90°C for 3 days. Neutral, acidic, and alkaline extraction solutions were diluted 25-fold, 50-fold, and 100-fold with 5% nitric acid, and mixed well. The extracted zinc concentration was measured by iCAP Q Inductively coupled plasma mass spectrometer (ICP-MS, Thermo Fisher Scientific). The configuration and the system and analytical parameters of ICP-MS device are described in Tables 2 and 3. The zinc concentration in the extraction solution was determined from the interpolation of the linear standard curve of 1 ppb, 5 ppb, 20 ppb, and 100 ppb zinc. The standard solutions of zinc at each concentration were prepared by diluting 1000 ppm zinc standard solution (VHG Labs PZNN-100) 100-fold with 5% nitric acid to prepare a stock solution of 10 ppm zinc, which was diluted with 5% nitric acid.

The measurement results are shown in Figure 1 and Table 4.

The zinc leaching capacity from the elastomer varied depending on the type of extraction solution. Elastomer A, Elastomer B, and Elastomer D showed almost no zinc leaching with any of the extraction solutions of neutral, acidic, or alkaline. In contrast, Elastomer C exhibited higher zinc leaching when the acidic or basic extraction solution was used, and Elastomer E exhibited higher zinc leaching when the acidic extract solution was used.

[Table 1]

<Table 1> Elastomer used in Example 1

| Name of Elastomer | Type of Elastomer |
|---|---|
| Elastomer A (BD260) | |
| Elastomer B (Stelmi 8550) | Thermoplastic elastomer (TPE) |
| Elastomer C (Stelmi 4800) | Polyisoprene type elastomer |
| Elastomer D (Sumitomo P-101A) | Chlorobutyl type elastomer |
| Elastomer E (Stelmi 4900) | Styrene-butadiene type elastomer |

[Table 2]

<Table 2> Configuration of ICP-MS device used in Example 1

| Component | Equipment, fixture |
|---|---|
| Device | Thermo iCAP Q |
| Autosampler | Cetax ASX 520 or ASX 560 |
| Spray chamber | Peltier-cooled Quartz Cycl onic spray chamber |
| Nebulizer | PFA Concentric Nebut izer |
| Torch | Quartz Torch 2.5 mm i.d. |
| Sample/Internal standard tube | Yellow/Orange 0.508mm i.d. |

[Table 3]

<Table 3> System and analytical parameters of ICP-MS device used in Example 1

| Parameter | Conditions |
|---|---|
| Power | 1550W |
| Cool Gas Flow | 14L/min |
| Aux. Flow | 0.8 L/min |
| Neb. Flow | 1L/min |
| Detection Mode | Kinetic Energy Discrimin ation (KED) |
| Collision Gas | Helium at 4. 0-5.0 mL/min |
| Dwell Time | 0.1s |

<Table 4> Concentration of zinc leached from 10 g of cut elastomer into 20 mL of extraction solution The extraction solutions used were water (neutral), 10 mM phosphoric acid solution (acidic), or 10 mM sodium hydroxide solution (alkaline). The extraction conditions were an incubation at 90°C for 3 days. As a control, an extraction solution subjected to incubation without elastomer was used.

[Table 4]

| Name of Elastomer | Zinc concentration ($\mu$g/mL) | | |
|---|---|---|---|
| | Neutral | Acidic | Alkaline |
| (Control) | <0.025 | <0.025 | <0.025 |
| Elastomer A | <0.025 | 0.066 | <0.025 |
| Elastomer B | 0.0425 | 0.105 | <0.025 |
| Elastomer C | 0.3875 | >10 | 4.473 |
| Elastomer D | <0.025 | 0.07 | <0.026 |
| Elastomer E | <0.025 | 2.291 | <0.025 |

EXAMPLE 2

Example 2: Leaching rate of zinc from elastomer under similar conditions to formulation storage conditions

Polyisoprene type elastomer (Elastomer C in Example 1) was used. The nine formulated solutions described in Table 5 were used. As the conditions for the formulated solutions, the effects of representative buffers (citric acid, phosphoric acid, histidine), pH conditions (5.0, 6.0, 7.0), stabilizer (arginine), pH adjusters (hydrochloric acid (HCl), sodium hydroxide (NaOH), aspartic acid), and surfactant (poloxamer 188), which are typically used for formulated solutions of biopharmaceuticals, were verified.

To a 20 mL vial (white, sulfur treated, MURASE GLASS Co., Ltd.), 10 mL of each formulated solution and an elastomer cut into 4 pieces were filled. The vial was plugged with a stopper (F10-F6W, Daikyo Seiko, Ltd.), capped, and incubated for 3, 7, or 14 days at 5°C, which was used as a sample. As a control, a vial to which only the formulated solution was added without elastomer and incubated in the same way as in the sample was used. The diagram showing the preparation of a sample and a control is shown in Figure 2. The metal ion concentration in the solution after incubation was measured by ICP-MS (Agilent 7700x, Agilent Technologies). Quantification was performed in a semi-quantitative mode in which a concentration was calculated from the concentration of metal ions contained in the tuning solution (Agilent 5185-5959, 1 $\mu$g/L Ce, Co, Li, Mg, Tl, and Y) and the theoretical response ratio of the measured element. The configuration and the system and analytical parameters of ICP-MS device are described in Tables 6 and 7. The leaching amount of each metal ion per surface area of the elastomer ($M_{metal}(\mu g/mm^2)$) was determined from the following expression:

$$[\text{Expression 3}]$$

$$M_{metal}(\mu g/mm^2) = (C_{sample} - C_{control}) \text{ (ppm) x } 10^{-3} \text{ x } 10 \text{ (mL/elastomer)}/700 \text{ (mm}^2/\text{elastomer)}$$

wherein, $C_{sample}$(ppm), $C_{control}$(ppm), 10 (mL/elastomer), and 700 (mm$^2$/elastomer) are metal ion concentration in sample, metal ion concentration in control, volume of extraction solution per elastomer, and approximate surface area per elastomer being cut into four, respectively.

The leaching amount of each metal ion ($M_{metal}(\mu g/mm^2)$) upon incubation at 5°C for 14 days is shown in Table 8. No leaching of metal ions other than zinc was detected in the conditions of all the formulated solutions. The citrate buffer (Formulation No. 1) and the phosphate buffer (Formulation No. 2) exhibited similar amounts of zinc leaching, while the histidine buffer (Formulation No. 4) exhibited about 10-fold zinc leaching compared to the citrate buffer (Formulation No. 1) and the phosphate buffer (Formulation No. 2). The effect of pH on the zinc leaching amount was not significant in the range of pH 5.0 to pH 7.0 (Formulation No. 3, Formulation No. 4, Formulation No. 5). The zinc leaching was suppressed by the addition of 75 mM arginine (Formulation No. 4, Formulation No. 6), but there was little change in the zinc leaching amount when arginine was added up to 150 mM (Formulation No. 6, Formulation No. 7). The use of aspartic acid as a pH adjusting agent instead of HCl promoted leaching of zinc (Formulation No. 7, Formulation No. 8). The addition of poloxamer 188 promoted leaching of zinc, but the effect was small (Formulation No. 8, Formulation No. 9). Among the investigated formulated solutions, Formulation No. 9 (20 mM histidine, 150 mM arginine, aspartic acid (q.s.), 0.5 mg/mL poloxamer 188, pH 6.0) was found to be most likely to cause leaching of zinc.

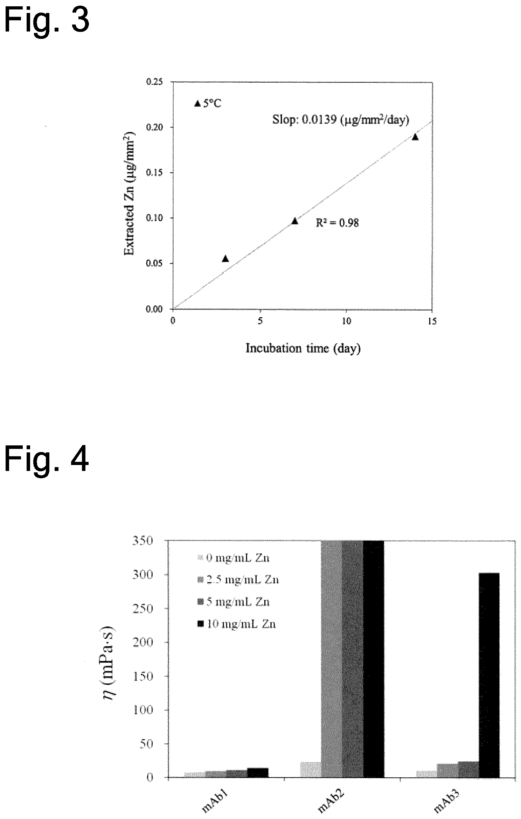

Then, the zinc leaching amount ($M_{zinc}(\mu g/mm^2)$) at 5°C was determined using Formulation No. 9 (20 mM histidine, 150 mM arginine, aspartic acid (q.s.), 0.5 mg/mL poloxamer 188, pH 6.0) at days 3, 7, and 14 (Table 9), and the zinc leaching rate ($k_{ext}(\mu g/mm^2/day)$) at 5°C per surface area of the elastomer was calculated from the determined zinc leaching amount to have a value of 0.0139 ($\mu g/mm^2/day$) (Figure 3). Using this zinc leaching rate value, and assuming that the cross-sectional area of the needle bevel to be 0.06 (mm$^2$) and the volume of the drug solution in the needle bevel to be 60 (nL), an expected zinc concentration ($C_{zinc}^{max}(mg/mL)$) when the drug solution in the needle bevel is in contact with the elastomer for 2 years at 5°C was determined from the following expression to be about 10 (mg/mL).

$$[\text{Expression 4}]$$

$$C_{zinc}^{max}(mg/mL) = 0.0139 \text{ (}\mu g/mm^2/day\text{) x } 730 \text{ (day) x } 0.06 \text{ (mm}^2\text{)}/0.06 \text{ (}\mu L\text{)} \approx 10 \text{ (mg/mL)}$$

Considering this calculated expected zinc concentration, the zinc spiking experiment in Example 3 was performed at a zinc concentration of 0 to 10 mg/mL.

[Table 5]

<Table 5> Formulated solution used in Example 2

| Formulation No. | Formulation conditions |
|---|---|
| Formulation No. 1 | 20 mM citric acid, NaOH (appropriate amount), pH 6.0 |
| Formulation No. 2 | 20 mM phosphoric acid, pH 6.0 |
| Formulation No. 3 | 20 mM histidine, HCl (appropriate amount), pH 5.0 |
| Formulation No. 4 | 20 mM histidine, HCl (appropriate amount), pH 6.0 |
| Formulation No. 5 | 20 mM histidine, HCl (appropriate amount), pH 7.0 |
| Formulation No. 6 | 20 mM histidine, 75 mM arginine, HCl (appropriate amount), pH 6.0 |
| Formulation No. 7 | 20 mM histidine, 150 mM arginine, HCl (appropriate amount), pH 6.0 |
| Formulation No. 8 | 20 mM histidine, 150 mM arginine, aspartic acid (appropriate amount), pH 6.0 |
| Formulation No. 9 | 20 mM histidine, 150 mM arginine, aspartic acid (appropriate amount), 0.5 mg/mL poloxamer 188, pH 6.0 |

[Table 6]

<Table 6> Configuration of ICP-MS device used in Example 2

| Component | Equipment, fixture |
|---|---|
| Device | Agilent 7700 x |
| Autosampler | G3160B |
| Spray chamber | Peltier-cooled Quartz Scot t spray chamber |
| Nebulizer | Glass Concentric Nebulizer |
| Torch | Quartz Torch 2.5mm i. d. |
| Sample/Internal standard tube | PFA 0.3 mm i. d . |

[Table 7]

<Table 7> System and analytical parameters of ICP-MS device used in Example 2

| Parameter | Conditions |
|---|---|
| Power | 1550 W |
| Cool Gas Flow | 15.0 L/min |
| Aux. Flow | 0.9 L/min |
| Neb. Flow | 1.07 L/min |
| Detection Mode | Collision Induced Dissoci ation (CID)/Kinetic Energy Discrimination (KED) |
| Collision Gas | Helium at 4.3 mL/min |
| Dwell Time | 0.3 s |

<Table 8> Leaching amount of metal ions per surface area of elastomer (polyisoprene type elastomer, Elastomer C described in Table 1) when stored at 5°C for 14 days ($M_{metal}(\mu g/mm^2)$)

[Table 8]

| Formulation No. | $M_{metal}$ ($\mu$g/mm$^2$) | | | | | | | | | | | | | | | |
|---|---|---|---|---|---|---|---|---|---|---|---|---|---|---|---|---|
| | Al | Ti | V | Cr | Mn | Fe | Co | Ni | Cu | Zn | As | Mo | Sn | Ba | Ce | Gd |
| No. 1 | <0.001 | | | | | | | | | 0.012 | <0.001 | | | | | |
| No. 2 | <0.001 | | | | | | | | | 0.010 | <0.001 | | | | | |
| No. 3 | <0.001 | | | | | | | | | 0.102 | <0.001 | | | | | |
| No. 4 | <0.001 | | | | | | | | | 0.131 | <0.001 | | | | | |
| No. 5 | <0.001 | | | | | | | | | 0.115 | <0.001 | | | | | |
| No. 6 | <0.001 | | | | | | | | | 0.060 | <0.001 | | | | | |
| No. 7 | <0.001 | | | | | | | | | 0.053 | <0.001 | | | | | |
| No. 8 | <0.001 | | | | | | | | | 0.146 | <0.001 | | | | | |
| No. 9 | <0.001 | | | | | | | | | 0.191 | <0.001 | | | | | |

<Table 9> Zinc leaching amount per surface area ($M_{zinc}$($\mu$g/mm$^2$)) of elastomer (polyisoprene type elastomer, Elastomer C described in Table 1) when stored at 5°C for 3, 7, and 14 days in Formulation No. 9 (20 mM histidine, 150 mM arginine, aspartic acid (q.s.), and 0.5 mg/mL poloxamer 188, pH 6.0).

[Table 9]

| Storage period | $M_{zinc}$ ($\mu$g/mm$^2$) |
|---|---|
| 3 days | 0.056 |
| 7 days | 0.098 |
| 14 days | 0.191 |

EXAMPLE 3

Example 3: Effect of Zinc on Viscosity of Antibody Solution

To clarify the physicochemical effect of leaching zinc from the elastomer of the needle shield on the monoclonal antibody (mAb) solution in the needle bevel, the effect of zinc on the viscosity of the three mAb solutions was investigated. Humanized IgG mAb1 (IgG1, tocilizumab, CAS: 375823-41-9), mAb2 (IgG1), and mAb3 (IgG2, satralizumab, RG6168) produced and purified by CHUGAI PHARMACEUTICAL CO., LTD. were used. Note that mAb2 and mAb3 are antibodies to which SMART technology has been applied, but mAb1 is an antibody to which SMART technology has not been applied. In mAb3, low pI technology has been further applied.

The viscosity of each mAb solution (180 mg/mL) was measured by an electromagnetic spinning method (EMS method) using an EMS viscometer (KYOTO ELECTRONICS MANUFACTURING CO., LTD.) at 25°C. Formulation No. 9 (20 mM histidine, 150 mM arginine, aspartic acid (q.s.), 0.5 mg/mL poloxamer 188, pH 6.0) in Table 5 was selected as the formulated solution, and the zinc concentration was 0, 2.5, 5, or 10 mg/mL (0, 5.2, 10.4, or 20.8 mg/mL as the zinc chloride concentration). The EMS viscometer is composed of a rotor equipped with a pair of permanent magnets, a brushless DC motor, a flash lamp, a CCD video camera, and a temperature regulator. In the EMS method, a liquid sample and an aluminum ball were placed in a glass tube, and the aluminum ball is rotated using a moment by a Lorentz force. The viscosity of the liquid sample can be calculated from the rotational speed of the aluminum ball measured with the flash lamp and the CCD video camera. A mAb solution and an aluminum ball with a diameter of 2 mm were placed in A glass tube with an inner diameter of 6.3 mm, and the viscosity was measured at a rotational speed of 1000 rpm. To prevent adsorption of the sample to the aluminum ball, the aluminum ball was previously treated by a silicon coating in a desiccator with a steam of dimethyldichlorosilane.

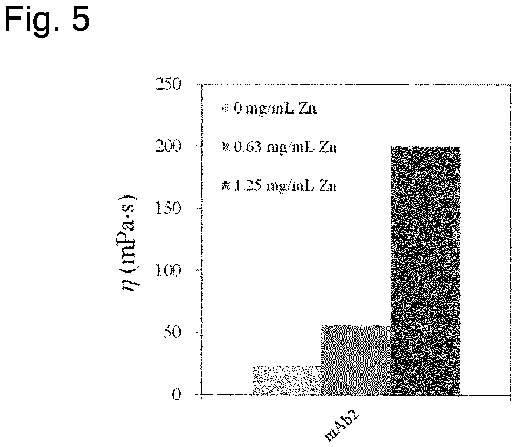

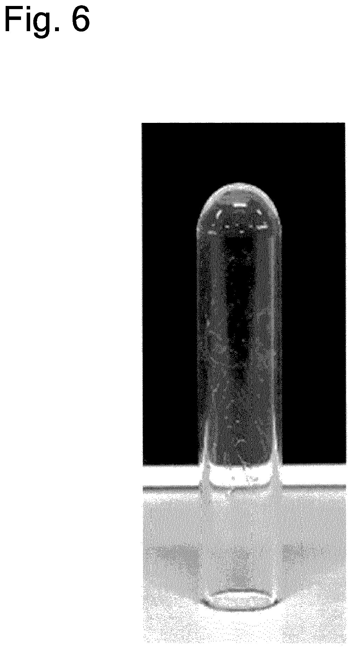

The reactivities with zinc were clearly different depending on the type of mAb (Table 10, Figure 4). In mAb1, only a slight viscosity increase was observed with zinc addition in the range of 0 to 10 mg/mL, while a dramatic viscosity increase was observed with zinc in mAb2 and mAb3. In mAb2, a clear viscosity increase was observed even at a zinc concentration of 0.63 mg/mL (Table 11, Figure 5), and gelation occurred at a zinc concentration of 2.5 mg/mL or more, indicating that the viscosity exceeded the measurable upper limit (8000 mPa·s) of the device. Figure 6 shows a photograph of the gelation of the mAb2 solution caused by the addition of 10 mg/mL zinc. In mAb3, the addition of 10 mg/mL zinc increased the viscosity about 27-fold (Table 10).

When an elastomer having a zinc leaching rate of 0.0139 $\mu$g/mm$^2$/day at 5°C per surface area is used, the zinc concentration in the needle bevel after 2 years at 5°C is expected to be about 10 mg/mL. However, as mentioned above, in the development of a staked-in needle prefilled syringe formulation of an antibody such as mAb2 that is highly reactive with zinc, it is necessary to keep the zinc concentration in the needle bevel after 2 years at 5°C to 0.63 mg/mL or less to avoid the risk of the needle clogging due to sudden viscosity increase caused by zinc. It is thus necessary to select, as a needle shield, an elastomer having a zinc leaching rate at 5°C per surface area of 0.88 ng/mm$^2$/day (= 0.0139 ($\mu$g/mm$^2$/day)/10 (mg/mL) x 0.63 (mg/mL)) or less. In addition, in the development of a staked-in needle prefilled syringe formulation of an antibody such as mAb3, the zinc concentration in the needle bevel after 2 years at 5°C should be suppressed to 5 mg/mL or less to avoid the risk of needle clogging due to sudden viscosity increase due to zinc. It is thus necessary to select, as a needle shield, an elastomer having a zinc leaching rate at 5°C per surface area of 7 ng/mm$^2$/day (= 0.0139 ($\mu$g/mm$^2$/day)/10 (mg/mL) x 5 (mg/mL)) or less.

<Table 10> Viscosities of mAb solutions (180 mg/ml mAb, 20 mM histidine, 150 mM arginine, aspartic acid (q.s.), 0.5 mg/ml poloxamer 188, pH 6.0) at zinc concentration of 0, 2.5, 5, 10 mg/ml at 25°C, respectively

[Table 10]

| mAb Type | Viscosity (mPa·s) | | | |
|---|---|---|---|---|
| | 0 mg/mL Zinc | 2.5 mg/mL Zinc | 5mg/mL Zinc | 10mg/mL Zinc |
| mAb1 | 8.11 | 9.55 | 11.10 | 14.30 |
| mAb2 | 23.67 | >8000 | >8000 | >8000 |
| mAb3 | 11.33 | 21.10 | 24.30 | 303.00 |

<Table 11> Viscosities of mAb2 solution (180 mg/ml mAb2, 20 mM histidine, 150 mM arginine, aspartic acid (q.s.), 0.5 mg/ml poloxamer 188, pH 6.0) of zinc concentration 0, 0.63, 1.25 mg/mL at 25°C, respectively

[Table 11]

| mAb Type | Viscosity (mPa·s) | | |
|---|---|---|---|
| | 0 m g/mL Zinc | 0.63mg/mL Zinc | 1.25mg/mL Zinc |
| mAb2 | 23.67 | 55.77 | 200 |

EXAMPLE 4

Example 4: Evaluation of Clogging after Storage

A 27G staked-in needle syringe (1 mL standard) fitted with a needle shield having a low zinc leaching capacity, i.e., a zinc leaching rate at 5°C per surface area of 0.88 ng/mm$^2$/day or less, or a zinc concentration in the needle bevel after 2 years at 5°C of 0.63 mg/mL or less, is filled aseptically with 0.9 mL of a solution (antibody of interest: 180 mg/mL, histidine: 20 mmol/L, arginine: 150 mmol/L, aspartic acid: q.s., poloxamer 188: 0.5 mg/mL, pH 6.0) of a conventional antibody without particular engineering technology or an engineered antibody (an antibody having one or more histidine residue pairs adjacent to each other within three residues in the CDR region), plugged with a stopper, and stored under specific storage conditions (storage at 40°C for 6 months, at 25°C for 12 months and 5°C for 24 months). Then, an evaluation of clogging is carried out.

In addition, a 27G staked-in needle syringe (1 mL standard) fitted with a needle shield having a zinc leaching capacity, i.e., a zinc leaching rate at 5°C per surface area of 0.0139 $\mu$g/mm$^2$/day or more, or a zinc concentration in the needle bevel after 2 years at 5°C of 10 mg/mL or more, similarly filled with 0.9 mL of either of the two antibody-containing solutions is used as a control.

Evaluation method for needle clogging

Each sample is set in a predetermined position on an autograph, then a discharge operation is performed on the staked-in injection needle syringe that is fixed to the autograph in advance. The load on the plunger stopper is measured at conditions of a discharge rate of 100 mm/min. The definition of needle clogging is a needle clogging that is determined as not discharging a sample at the time of discharge or exhibiting an abnormally high value of load stress compared to that in a conventional discharge.

Evaluation results

It is confirmed that the frequency of clogging in the engineered antibody is higher when the occurrence of needle clogging (clogging) under specific storage conditions (6 months at 40°C, 12 months at 25°C, up to 24 months at 5°C) is compared between a conventional antibody and the engineered antibody using the needle shield having a zinc leaching capacity. Furthermore, it is confirmed that the frequency of clogging in the engineered antibody is reduced by using a needle shield having a low zinc leaching capacity compared to using a needle shield having a zinc leaching capacity.

EXAMPLE 5

Example 5: Prefilled syringe formulation of mAb3 (IgG2, satralizumab, RG6168)

Based on the results shown in Example 3, an elastomer having a zinc leaching rate of 7 ng/mm$^2$/day or less at 5°C per surface area was selected as the needle shield for the staked-in needle prefilled syringe formulation of the mAb3 antibody.

A 27G staked-in needle syringe (1 mL standard) was filled with 1 mL of an aqueous solution containing satralizumab (genetically modified) (satralizumab (genetically modified): 120 mg/mL, histidine: 20 mmol/L, arginine: 150 mmol/L, aspartic acid: q.s., poloxamer 188: 0.5 mg/mL, pH 6.0). The material of the needle shield was Elastomer D of Example 1.

**Claims**

1.   A prefilled syringe formulation comprising: a staked-in needle syringe filled with a drug solution; and an elastomeric needle shield, wherein

   the drug solution contains one or more antibodies,
   the one or more antibodies have a total number of histidine, aspartic acid, and glutamic acid present in a CDR region of 6 or more, and
   the elastomeric needle shield has a low zinc leaching capacity to an antibody-containing solution.

2.   The prefilled syringe formulation according to claim 1, comprising: a staked-in needle syringe filled with a drug solution; and an elastomeric needle shield, wherein

   the drug solution contains one or more antibodies,
   the one or more antibodies have one or more histidine residue pairs that are adjacent to each other within three residues in a CDR region, and

   the elastomeric needle shield has a low zinc leaching capacity to an antibody-containing solution.

3.   The prefilled syringe formulation according to claim 1 or 2, wherein the one or more antibodies are antibodies that bind to an antigen with higher binding activity at a neutral pH than at an acidic pH.

4.   The prefilled syringe formulation according to claim 3, wherein the one or more antibodies are antibodies having a value of KD (pH 5.8)/(pH 7.4), a ratio of KD at pH 5.8 to KD at pH 7.4 for an antigen, of 2 or more.

5.   The prefilled syringe formulation according to any one of claims 1 to 4, wherein the one or more antibodies have a substitution of at least one amino acid with histidine or an insertion of at least one histidine.

6.   The prefilled syringe formulation according to any one of claims 1 to 5, wherein the one or more antibodies are antibodies in which a charge of at least one amino acid residue that can be exposed to a surface of a CDR region is engineered.

7.   The prefilled syringe formulation according to any one of claims 1 to 6, wherein the low zinc leaching capacity is evaluated by a zinc leaching rate at 5°C per surface area of the needle shield in a buffer at pH 6.0, containing 20 mM histidine, 150 mM arginine, aspartic acid (q.s.), and 0.5 mg/mL poloxamer 188.

8. The prefilled syringe formulation according to any one of claims 1 to 7, wherein the low zinc leaching capacity is evaluated by an expected zinc concentration after 2 years at 5°C in a needle bevel obtained from a zinc leaching rate at 5°C per surface area of the needle shield.

9. The prefilled syringe formulation according to any one of claims 1 to 8, wherein a material of the needle shield is selected from the group consisting of a thermoplastic elastomer and a thermosetting elastomer.

10. A method of suppressing a viscosity increase and/or a needle clogging of a prefilled syringe comprising: a staked-in needle syringe filled with a drug solution; and an elastomeric needle shield, the method **characterized in that**

the drug solution contains one or more antibodies having a total number of histidine, aspartic acid, and glutamic acid present in a CDR region of 6 or more, and
the method comprises selecting a material of the needle shield so that the needle shield has a low zinc leaching capacity to the drug solution, wherein
the material of the needle shield is selected from the group consisting of a thermoplastic elastomer and a thermosetting elastomer.

11. The method according to claim 10, wherein

the drug solution contains one or more antibodies having one or more histidine residue pairs that are adjacent to each other within three residues in a CDR region, and
the method comprises selecting a material of the needle shield so that the needle shield has a low zinc leaching capacity to the drug solution.

12. The method according to claim 10 or 11, wherein the one or more antibodies are antibodies that bind to an antigen with higher binding activity at a neutral pH than at an acidic pH.

13. A syringe comprising: a longitudinal body having an interior in which an antibody-containing solution is placed; a needle connected to one end in a longitudinal direction of the longitudinal body; and a needle cap enclosing the needle, wherein

the needle cap has an elastomeric needle shield, and
the needle shield has a low zinc leaching capacity to a solution containing one or more antibodies having a total number of histidine, aspartic acid, and glutamic acid present in a CDR region of 6 or more.

14. The syringe according to claim 13, wherein the low zinc leaching capacity is evaluated by a zinc leaching rate at 5°C per surface area of the needle shield.

15. A method for producing a staked-in needle syringe formulation, comprising:

providing a drug solution containing one or more antibodies having a total number of histidine, aspartic acid, and glutamic acid present in a CDR region of 6 or more;
providing a needle shield having a low zinc leaching capacity to the drug solution; and
filling a staked-in needle syringe having the needle shield with the drug solution, wherein
the needle shield has a zinc leaching rate at 5°C per surface area of 0.88 ng/mm$^2$/day or less.

# Fig. 1

# Fig. 2

# Fig. 3

# Fig. 4

# Fig. 5

Fig. 6

## INTERNATIONAL SEARCH REPORT

| International application No. |
|---|
| PCT/JP2021/027663 |

**A. CLASSIFICATION OF SUBJECT MATTER**
Int. Cl. A61K39/395(2006.01)i, C07K16/00(2006.01)i, A61K9/08(2006.01)i,
A61M5/32(2006.01)i, C12N15/13(2006.01)i
FI: A61K39/395 M, A61M5/32 500, A61K39/395 H, C12N15/13, C07K16/00, A61K9/08 ZNA

According to International Patent Classification (IPC) or to both national classification and IPC

**B. FIELDS SEARCHED**

Minimum documentation searched (classification system followed by classification symbols)
Int. Cl. A61K39/395, C07K16/00, A61K9/08, A61M5/32, C12N15/13

Documentation searched other than minimum documentation to the extent that such documents are included in the fields searched
Published examined utility model applications of Japan      1922-1996
Published unexamined utility model applications of Japan   1971-2021
Registered utility model specifications of Japan          1996-2021
Published registered utility model applications of Japan  1994-2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS
(STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2017-538463 A (F HOFFMANN-LA ROCHE AG) 28 December 2017 (2017-12-28), claims 1-3, 7, 8, 12, 13, abstract, paragraphs [0013], [0014], [0027], examples | 1-15 |
| Y | JP 2017-536800 A (CHUGAI PHARMACEUTICAL CO., LTD.) 14 December 2017 (2017-12-14), claims, sequence listing, paragraph [0601] | 1-15 |
| Y | WO 2009/041621 A1 (CHUGAI PHARMACEUTICAL CO., LTD.) 02 April 2009 (2009-04-02), claims, sequence listing | 1-15 |
| A | WO 2016/068333 A1 (CHUGAI PHARMACEUTICAL CO., LTD.) 06 May 2016 (2016-05-06), claims | 1-15 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13.08.2021 | 24.08.2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| | International application No. |
|---|---|
| | PCT/JP2021/027663 |

| Patent Documents referred to in the Report | Publication Date | Patent Family | Publication Date |
|---|---|---|---|
| JP 2017-538463 A | 28.12.2017 | US 2017/0333641 A1<br>claims 1-3, 7, 8, 12, 13, abstract, paragraphs [0013], [0014], [0027], examples<br>EP 3212252 A1<br>KR 10-2017-0076726 A<br>CN 107148287 A | |
| JP 2017-536800 A | 14.12.2017 | US 2016/0229908 A1<br>claims, paragraph [0919], sequence listing<br>EP 3253778 A1<br>CN 107108729 A<br>KR 10-2017-0110129 A | |
| WO 2009/041621 A1 | 02.04.2009 | US 2011/0245473 A1<br>claims, sequence listing<br>EP 2206775 A1<br>CN 101939425 A<br>KR 10-2010-0061748 A | |
| WO 2016/068333 A1 | 06.05.2016 | US 2018/0133375 A1<br>claims<br>EP 3213786 A1<br>KR 10-2017-0078707 A<br>TW 201630630 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016068333 A
- WO 2017538463 A
- WO 2016066821 A
- WO 2009125825 A
- US 20110111406 A
- WO 2009041643 A
- US 20100239577 A
- US 20100239577 A1
- EP 239400 A
- WO 9602576 A
- WO 0941613 A
- JP 1059878 B
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- JP 6266164 B
- WO 2009041621 A
- WO 2009072604 A

**Non-patent literature cited in the description**

- **KOHLER. G. ; MILSTEIN, C.** *Methods Enzymol.,* 1981, vol. 73, 3-46
- **CARL, A. K. BORREBAECK ; JAMES, W. LAR-RICK.** therapeutic monoclonal antibodies. Macmillan publishers Ltd, 1990
- **SATO, K. et al.** *Cancer Res.,* 1993, vol. 53, 851-856
- **TSURUSHITA N ; HINTON PR ; KUMAR S.** Design of Humanized Antibodies: from anti-Tac to Zenapax. *Methods.,* May 2005, vol. 36 (1), 69-83
- **RAJPAL A ; BEYAZ N ; HABER L ; CAPPUCCILLI G ; YEE H ; BHATT RR ; TAKEUCHI T ; LERNER RA ; CREA R.** A general method for greatly improving the affinity of antibodies by using combinatorial libraries. *Proc Natl Acad Sci USA.,* 14 June 2005, vol. 102 (24), 8466-71
- **EWERT S ; HONEGGER A ; PLUCKTHUN A.** Stability improvement of antibodies for extracellular and intracellular applications: CDR grafting to stable frameworks and structure-based framework engineering. *Methods.,* 03 October 2004, vol. 4 (2), 184-99
- **KIM SJ ; PARK Y ; HONG HJ.** Antibody engineering for the development of the therapeutic antibodies. *Mol Cells.,* 31 August 2005, vol. 20 (1), 17-29
- **HINTON PR ; XIONG JM ; JOHLFS MG ; TANG MT ; KELLER S ; TSURUSHITA N.** An engineered human IgG1 antibody with longer serum half-life. *J Immunol.,* 01 January 2006, vol. 176 (1), 346-56
- **GHETIE V ; POPOV S ; BORVAK J ; RADU C ; MATESOI D ; MEDESAN C ; OBER RJ ; WARD ES.** Increasing the serum persistence of an IgG fragment by random mutagenesis. *Nat Biotechnol.,* July 1997, vol. 15 (7), 637-40
- *Farmaco.,* 30 August 1999, vol. 54 (8), 497-516
- *Cancer J.,* May 2008, vol. 14 (3), 154-69